# EUROPEAN PATENT SPECIFICATION

(11) **EP 2 965 088 B1**
(45) Date of publication and mention of the grant of the patent: **05.12.2018**
(21) Application number: 14707415.7
(22) Date of filing: 03.03.2014
(51) Int. Cl.: G01N 27/62, G01N 21/49, G01N 33/68

(54) **PROGNOSTIC MARKER TO DETERMINE THE RISK FOR EARLY-ONSET PREECLAMPSIA**
PROGNOSTISCHE MARKER ZUR FESTSTELLUNG DES RISIKOS FRÜH EINSETZENDER PRÄEKLAMPSIE
MARQUEUR PRONOSTIQUE POUR DÉTERMINER LE RISQUE DE PRÉÉCLAMPSIE PRÉCOCE

(30) Priority: 04.03.2013 US 201361772501 P; 10.06.2013 EP 13171331
(43) Date of publication of application: 13.01.2016
(73) Proprietor: IQ Products B.V., 9727 DL Groningen (NL)
(72) Inventor: SCHUITEMAKER, Joost, Henric, Nicolaas, 9356 TJ Tolbert (NL)
(74) Representative: Ellens, Andries
(86) International application number: PCT/EP2014/054053
(87) International publication number: WO 2014/135488

(56) References cited:
- EP-A1- 2 490 027
- WO-A1-2009/066821
- WO-A2-2004/003172
- WO-A2-2004/021978
- SARAH CROSS ET AL: "Endocan (ESM-1): a novel soluble endothelial cell injury marker in preeclampsia (PE) and intrauterine growth restriction (IUGR)", 33RD ANNUAL MEETING/PREGNANCY MEETING OF THE SOCIETY-FOR-MATERNAL-FETAL-MEDICINE (SMFM); SAN FRANCISCO, CA, USA, vol. 208, 20 December 2012 (2012-12-20), page S276, XP055121155, DOI: 1
- GRILL S ET AL: "Potential markers of preeclampsia - A review", REPRODUCTIVE BIOLOGY AND ENDOCRINOLOGY, BIOMED CENTRAL LTD, GB, vol. 7, no. 70, 14 July 2009 (2009-07-14), pages 1-14, XP002599715, ISSN: 1477-7827, DOI: 10.1186/1477-7827-7-70
- LIM J H ET AL: "Effective Prediction of Preeclampsia by a Combined Ratio of Angiogenesis-Related Factors", OBSTETRICS AND GYNECOLOGY, LIPPINCOTT WILLIAMS & WILKINS, US, vol. 111, no. 6, 1 June 2008 (2008-06-01), pages 1403-1409, XP002688316, ISSN: 0029-7844, DOI: 10.1097/AOG.0B013E3181719B7A

## Description

### FIELD OF THE INVENTION

The invention relates to the prediction, especially in an early stage of gestation, and diagnosis, in the later stages of gestation, of preeclampsia.

### BACKGROUND OF THE INVENTION

Methods for diagnosing preeclampsia are known in the art. WO2013071703 for instance describes a means for rapidly detecting Adipsin for diagnosing preeclampsia comprises a water-adsorbing pad, a nitrocellulose membrane, a gold labeling pad and a sampling pad, all of which are successively joined from the top down and fixed in the base plate. The gold-labeling pad is partly overlapped by the sampling pad. A detecting line coated by rabbit anti-human Adipsin polyclonal antibody, or goat anti-human Adipsin polyclonal antibody or mouse anti-human Adipsin polyclonal antibody and a controlling line coated by goat anti-mouse IgG polyclonal antibody are provided on the nitrocellulose membrane. The detecting line is located downstream and spaced from the controlling line. The gold-labeling pad is made of water adsorbing material, and coated by gold labeled mouse anti-human Adispin monoclonal antibody. A test kit for rapidly detecting Adipsin for diagnosing preeclampsia comprises an outer house and the said means therein. The method for preparing the said detecting means comprise: 1) coating of the detecting line and the controlling line; 2) preparing the gold labeling pad; 3) combination.

WO2014001244 describes a method for diagnosing whether a pregnant subject is not at risk for Preeclampsia within a short window of time comprising a) determining the amount of at least one angiogenesis biomarker selected from the group consisting of sFlt-1, Endoglin and P1GF in a sample of said subject, and b) comparing the amount with a reference, whereby a subject being not at risk for developing preeclampsia within a short period of time is diagnosed if the amount is identical or decreased compared to the reference in the cases of sFlt-1 and Endoglin and identical or increased in the case of P1GF, wherein said reference allows for making the diagnosis with a negative predictive value of at least about 98%. US20050170444 describes methods for diagnosing Preeclampsia and Eclampsia or a propensity to develop Preeclampsia or Eclampsia by detecting the levels of placental growth factor in a subject.

Sara Cross et al. describe in "Endocan (ESM-1): a novel soluble endothelial cell injury marker in preeclampsia (PE) and intrauterine growth restriction (IUGR)" (33rd Annual Meeting / Pregnancy meeting of the society for maternal fetal medicine (SMFM), San Francisco, CA, USA; vol. 208, 20 December 2012, page S276) that women with preterm sPE have elevated serum ESM-1 levels, likely reflecting advanced degree of endothelial activation and injury at earlier GA. Enhanced expression at sites of major placental damage suggest ESM-1 may play a role in the pathophysiology of trophoblast injury in IUGR.

WO2004021978 describes antisense compounds, compositions, and methods for modulating the expression of Endothelial Specific Molecule-1 (ESM-1). The compositions comprise antisense compounds, particularly antisense oligonucleotides, targeted to nucleic acids encoding ESM-1. Methods of using these compounds for modulation of ESM-1 expression and for treatment of diseases associated with expression of ESM-1 are decribed.

WO2004003172 relates to a nucleic acid and its encoded polypeptide ESM-1, whose expression is modulated in angiogenesis and oncogenesis. The application also relates to antibodies having specificity for said polypeptide, antisense molecules, and to methods useful for treating or modulating angiogenesis in mammals in need of such biological effect.

### SUMMARY OF THE INVENTION

Preeclampsia is a syndrome which is characterized by high blood pressure (>140/90) and significant amounts of protein in the urine (>300 mg/24 hrs urine) predominantly during the last weeks of pregnancy. Two forms are clinically recognized; the early-onset preeclampsia, which appears before the 34th week gestational age (GA), and the late-onset preeclampsia, which appears after the 34th week of GA. Untreated the condition might result in, for mother and child, long term effects and even a life-threatening situation. And although some women benefit from a low dose Aspirin supplementation or can be stabilized by intravenous magnesium sulfate, the best treatments for Eclampsia or advancing preeclampsia are abortion or delivery. This makes monitoring for and treatment of the early-onset preeclampsia even more important since this increases the chances for mother and child, simply because of the fact that delivery should be delayed as long as possible. A lot of research is done to find the mechanism that results to this syndrome. In this research several factors are found that might play a role in the etiology of the disease. The maternal immune system and the fact whether gestational immune tolerance is build effectively seems to play an important part. The current understanding of the syndrome is that it is a two-stage process. The initial lack of gestational immune tolerance of the placental cytotrophoblasts may lead to inadequately remodeled spiral arteries and a shallow implantation, which in turn lead to downstream hypoxia. This hypoxia of the placenta seems to be an important factor, since it is followed by the release of soluble factors in the maternal circulation. Some soluble factors, like soluble Fms-like tyrosine kinase-1 (sFlt-1 or sVEGFR-1) and soluble Endoglin (sEng), have been described as markers which are differentially expressed in preeclamptic pregnancies after week 20 of gestation compared to healthy pregnancies. These factors currently are used for screening during pregnancy and concentrations give indications of the severity of the disease.

A disadvantage of present solutions to detect Preeclampsia or Eclampsia and other pregnancy related syndromes such as Hemolysis Elevated Liver enzymes and Low Platelets (HELLP), and Intra Uterine Growth Restriction (IUGR) is that those syndromes are detectable late in the pregnancy. This prevent taking measure early during pregnancy to prevent the occurrence or reduce the severity of the syndrome. Knowing early during pregnancy that a pregnant woman will develop such syndrome also reduced unnecessary preventive measures, such as e.g. the standard use of aspirin. The subject herein is especially a woman.

In 1996 a new marker for endothelial cells was discovered by Lassalle et al. (J. Biol. Chem. 1996, 271:20458-20464). This proteoglycan of 50 kD was called endothelial cell-specific molecule 1 (ESM-1) and is also known as Endocan. The expression and secretion of ESM-1 is upregulated by pro-inflammatory molecules, such as TNF alpha, and in the presence of pro-angiogenic factors such as VEGF. The molecule has been described to be upregulated in inflammatory conditions like sepsis, but also in cancer.

As indicated above, ESM-1 plays a role in the regulation of angiogenesis and is released by activated endothelial cells. It was tested whether ESM-1 is increased in preeclampsia (PE). Plasma samples from high risk pregnancies divided in 23 healthy (CON), 11 severe early-onset PE (SE) and 7 severe late-onset PE (SL) pregnancies were collected at regular intervals between week 8 and birth. ESM-1 was measured by ELISA. Between GA week 24 and birth, ESM-1 concentrations were significantly increased in both early and late preeclampsia compared to controls (Mann Whitney, p<0.05). Surprisingly, the concentration of ESM-1 also differed between the three groups at weeks 12 and 16. The ESM-1 concentration of healthy pregnancies (mean±SEM:1857±861 pg/ml) and those that developed severe late-onset PE (1298±371 pg/ml) are comparable, but in those pregnancies that develop severe early-onset PE, the concentration (410±355 pg/ml) is significantly lower as compared with healthy pregnancies. Hence, ESM-1 concentrations are increased during early and late severe preeclampsia, which may be due to endothelial cell activation in these conditions. Surprisingly, since ESM-1 is decreased at 12-16 weeks in patients that later on develop early onset severe PE, it might be a prognostic marker which can determine the risk of women to develop severe early-onset PE already as early as 1-20 weeks, such as 8-20 weeks, especially 8-16 weeks, even more especially 12 to 16 weeks of gestation.

Hence, in a first aspect, the invention provides a method for diagnosing whether a pregnant subject has, or is having a predisposition for the development of, preeclampsia, eclampsia, or HELLP, or not, said method comprising the determination of the amount of ESM-1 in a sample of said subject. Especially, the invention provides in a first aspect an (in vitro) method for identifying from a biological sample from a pregnant subject a pregnancy related syndrome selected from the group consisting of Preeclampsia, Eclampsia, Hemolysis Elevated Liver enzymes and Low Platelets (HELLP), the method including (i) (using an assay specific to ESM-1 and) measuring the amount of ESM-1 in the biological sample, wherein the biological sample (especially a biological fluid sample) is from the pregnant subject being in any one week of gestation between week 9-16, even more especially week 12-16; and (ii) comparing said amount of ESM-1 to a reference value, as is further defined in the accompanying claims. The method, as indicated above, may especially further comprise (iii) identifying the subject as being likely to have or develop the pregnancy related syndrome based on a comparison of the amount of ESM-1 to the reference value. With the present invention, already before week 24, even before week 20, the likeliness of developing such syndrome or even the presence may be detected. Hence, in a very early stage, a treatment or prevention therapy may be started. Herein, the phrase "measuring the amount of ESM-1" may also relate to measuring an equivalent of ESM-1 or measuring a metabolite of ESM-1. In an embodiment said ESM-1 is the level of free ESM-1, bound ESM-1, a metabolite of ESM-1 or total ESM-1. Especially, said amount of ESM-1 is the level of free ESM-1 in the biological sample. The reference value may especially be a reference value measured in a sample from a woman having a healthy pregnancy. Especially, a healthy pregnancy relates to a pregnancy of the subject that does not have or develops any of the herein indicated pregnancy related syndromes (during the entire pregnancy). This may especially indicate that the reference value is such ESM-1 value of a sample of a pregnant subject, which later appeared not to develop or have such pregnancy related syndrome. Such reference value may especially be a mean value from a group of pregnant subjects that appeared all not to have or develop such pregnancy related syndrome. As indicated above (and also below), the striking observation was done hat those pregnant subjects that had already the pregnancy related syndrome or later appeared to develop such syndrome had substantial lower ESM-1 values in biological samples of these subjects in taken in week 20 (after gestation) or earlier. Hence, in a specific embodiment the method further comprises (iii) identifying the subject as being likely to have or develop the pregnancy related syndrome based on a comparison of the amount of ESM-1 to the reference value when said amount of ESM-1 is smaller than the ESM-1 reference value. Hence, especially the reference value is the amount of ESM-1 in biological samples of pregnant subjects having a healthy pregnancy. Especially, the reference values may include a single reference value for a specific gestation period or a plurality of reference values for a plurality of periods within the gestation period. Especially, the measured ESM-1 value is compared to a reference value indicative for the time of gestation at which the biological fluid sample of the pregnant subject is taken. For instance, there may be specific reference values for week 8-12 and reference values for week 12-16, etc. Optionally, there may be different reference values for different types of samples. The reference value may be a number but may also be a color, a color intensity or lack thereof, be an amount of radioactivity, or an amount of light or lack thereof, as is known in the art.

Especially, the sample is a fluid sample, i.e. a biological fluid sample. In an embodiment, said sample is a blood, plasma, serum, urine or saliva from the subject. Especially, said biological sample comprises a blood sample, a plasma sample, or a serum sample from said pregnant subject. However, the sample may also include cerebrospinal fluid (CSF) or other biological fluid. Optionally, the biological sample may include a placenta biopt. Even more especially, said biological sample is from a pregnant subject being in any one week of gestation between weeks 12 to 16 of gestation. In yet even a more specific embodiment, said biological sample comprises a plasma example and the pregnant subject is evaluated to have the pregnancy related syndrome when the ESM-1 value is in the range of 410±355 pg/ml.

In a specific embodiment, the amount of ESM-1 of the biological sample of the pregnant subject is indicative of the pregnancy related syndrome when the amount is equal to or less than 80%, especially equal to or less than 70%, especially equal to or less than 60%, such as equal to or less than 50%, like even more especially equal to or less than 40%, of the amount of ESM-1 of biological samples of pregnant subjects having a healthy pregnancy. Of course, comparisons are made between samples taken in the same period of pregnancy, such as in the gestation period of in weeks 1-20, like weeks 12-16. Hence, the amount of ESM-1 of the biological sample of the pregnant subject may be indicative of the pregnancy related syndrome when the amount is equal to or less than 80%, especially equal to or less than 70%, especially equal to or less than 60%, such as equal to or less than 50%, like even more especially equal to or less than 40%, of the amount of ESM-1 of biological samples of pregnant subjects having a healthy pregnancy (wherein the biological sample of the pregnant subject and the reference value relate to samples in the same week (especially selected from week 1-2) of gestation.

Especially, the assay is an antibody based assay, i.e. the assay uses an antibody to detect the ESM-1 protein (or equivalent or metabolite). In a specific embodiment, the assay comprises an ELISA (Enzyme-linked immunosorbent assay) assay, a turbidimetric assay, radio immunosorbent assay (RIST), a radioimmunoassay (RIA), a direct or indirect immunofluorescence assay, a particle gel immuno assay (PaGIA), or a lateral flow assay. Even more especially, the assay comprises an ELISA. Alternatively or additionally, the assay comprises a lateral flow assay. Alternatively or additionally, the assay comprises an enzymatic assay, a colorimetric assay, or a luminescent assay. The measurements may alternatively or additionally also include mass spectrometry measurements, especially of metabolites of ESM-1. Hence, in an embodiment measuring the amount of ESM-1 in the biological sample includes using mass spectrometry (MS).

In yet a further embodiment, not the ESM-1 is directly measured but RNA coding for ESM-1 is detected, i.e. especially the amount of RNA coding for ESM-1 is detected. Hence, in a specific embodiment measuring the amount of ESM-1 in the biological sample comprises a method to do a quantification of RNA coding for ESM-1 in cells or cell-free RNA in the biological sample, especially a fluid (i.e. a liquid). In yet a further embodiment, the invention involves a method wherein measuring the amount of ESM-1 in the biological sample comprises a method to quantify the amount of protein via the amount of RNA coding for ESM-1. For instance, in an embodiment said quantification is done via RT-PCR, PCR, hybridization or other means to determine the amount of nucleotides. In addition to the ESM-1 marker, also one or more other markers may be used. The absolute values thereof may be compared to absolute reference values. However, alternatively or additionally, a ratio between the ESM-1 marker and the other marker may be used to be compared with a reference ratio. For instance, in a specific embodiment, the method may further comprise (iv) using (selecting) a secondary marker selected from the group consisting of soluble Fms-like tyrosine kinase-1 (sFlt1), Vascular Endothelial Growth Factor (VEGF), Placental Growth Factor (PlGF), Hepatocyte Growth Factor (HGF), soluble Endoglin, placental protein 13 (pp-13), Pregnancy- associated Plasma Protein A (PAPP-A) and Growth Differentiation Factor 15 (GDF-15), (v) using an assay specific for the secondary marker and measuring the amount of the secondary marker in the biological sample, and optionally comparing said amount of said secondary marker to a secondary marker reference value. This may further enhance certainty of the identification process. Alternative or additional to the above indicated secondary marker, also one or more or pikachurin and a hemopexin may be chosen. Pikachurin, also known as agrin-like protein (AGRINL) and EGF-like, fibronectin type-III and laminin G-like domain-containing protein (EGFLAM), is a protein that in humans is encoded by the EGFLAM gene. Hemopexin (or haemopexin or HPX), also known as beta-lB-glycoprotein is a protein that in humans is encoded by the HPX gene and belongs to hemopexin family of proteins.

In a further specific embodiment, the method may further comprise identifying the subject as being likely to have or develop the pregnancy related syndrome based on a comparison of the amount of ESM-1 to a reference value, and based on a comparison of the amount of said one or more secondary markers with the corresponding secondary marker reference value. Alternatively or additionally, in a specific embodiment, the method may further comprise determining a ratio of the amount of ESM-1 to the amount of the secondary marker, selecting a ratio reference value, and comparing the ratio with the ratio reference value. Hence, in yet a further specific embodiment, the method may further comprise identifying the subject as being likely to have or develop the pregnancy related syndrome based on a comparison of the amount of ESM-1 to a reference value, and based on a comparison of the ratio of the amount of ESM-1 to the amount of the secondary marker to the ratio reference value.

Alternatively or additionally, the method may include a multi-stage process, wherein samples taken at different times, like before week 12 and after week 16, or before week 20, and after week 20, etc., may be taken and may be used to identify whether or not the pregnant subject has is will likely develop the pregnancy related syndrome. Hence, in a specific embodiment, the method may (further) comprise providing a first biological sample from a subject extracted on a first occasion, and providing a second biological sample from the subject extracted on a second occasion, using an assay specific to ESM-1, measuring the amount of ESM-1 in the first biological sample, constituting a first amount, using an assay specific to ESM-1, measuring the amount of ESM-1 in the second biological sample, and constituting a second amount. The second occasion is especially later in time than the first occasion. At least the first sample is especially taken in week 20 of gestation or earlier (like 12-16). Further, the method and use, as well as the application of the kit may include extracting one biological sample, two biological samples, but also more than two biological samples. Especially, these are taken at different times, such as with one or more days in between, or one or more weeks in between. Especially in a specific embodiment, the method may further comprise identifying the subject as being likely to have or develop the pregnancy related syndrome based on a comparison of the first amount and the second amount.

In a specific embodiment, the method may further comprise the step of uterine artery Doppler screening. This screening may advantageously give additional information about the placentation. Poor placentation is known to be an additional indication for the risk of the development of pregnancy related problems.

In yet a further aspect, the invention also provides a device for identifying a pregnant subject being in any one of week 9-16 of gestation that is likely to have or develop a pregnancy related syndrome selected from the group consisting of Preeclampsia, Eclampsia, and HELLP, said device comprising: (a) an analyzing unit comprising a detection agent for ESM-1, for instance being an antibody, a (recombinant) receptor for ESM-1 or an aptamer, which allows the determination of the amount of ESM-1; (b) an evaluation unit comprising a data processor having implemented necessary algorithms for comparing the amount of ESM-1 determined with a reference value stored in a database in order to determine whether a pregnant subject is likely to have or develop preeclampsia, eclampsia, and/or HELLP. Especially, in an embodiment the device may further comprise an analyzing unit comprising a detection agent for sFlt1, VEGF, PlGF, HGF, sEndoglin, pp-13, PAPP-A or GDF-15, which allows the determination of the amount of sFlt1, VEGF, P1GF, HGF, sEndoglin, pp-13, PAPP-A or GDF-15, and an evaluation unit comprising a data processor having implemented necessary algorithms for comparing the amount of sFlt1, VEGF, PlGF, HGF, sEndoglin, pp-13, PAPP-A or GDF-15 determined with reference values stored in a database and calculate the ratio between the measured markers in order to determine whether a pregnant subject is likely to have or develop preeclampsia, eclampsia, and/or HELLP. As will be clear to a person skilled in the art, the analyzing units and the evaluation units, ESM-1 and one ore more of the other markers, may optionally be a single analyzing unit and/or a single evaluation unit, respectively. Especially, in yet a further embodiment the evaluation unit is also capable of giving therapeutic recommendations. Alternative or additional to the above indicated secondary marker, also one or more or pikachurin and a hemopexin may be chosen.

In yet a further aspect, the invention also provides the use of an evaluation of the amount of ESM-1 in an extracted biological sample from a pregnant subject being in any one of week 9-16 of gestation for identifying whether the pregnant subject is likely to have or develop a pregnancy related syndrome selected from the group consisting of Preeclampsia, Eclampsia, and HELLP as is further defined in the accompanying claims. Even more especially, the invention provides the in vitro use of an extracted biological sample from a pregnant subject being in any one of week 9-16 of gestation for identifying whether the pregnant subject is likely to have or develop a pregnancy related syndrome selected from the group consisting of Preeclampsia, Eclampsia, and HELLP, the use comprising: (a) using an assay specific to ESM-1, measuring the amount of ESM-1 in the biological sample; (b) comparing said amount of ESM-1 to a reference value; and (c) identifying the subject as being likely to have or develop the pregnancy related syndrome, as further defined in the accompanying claims..

In yet a further aspect, the invention provides a kit for identifying whether a pregnant subject being in any one of week 9-16 of gestation is likely to have or develop a pregnancy related syndrome selected from the group consisting of Preeclampsia, Eclampsia, and HELLP, said kit comprising: (a) an analyzing unit comprising a detection agent for ESM-1; (b) an evaluation unit for determining whether a pregnant subject is likely to have or develop the pregnancy related syndrome, based on a result provided by the analyzing unit, as is further defined in the accompanying claims. Especially, the invention provides an embodiment of the kit further comprising a manual or a reference to a manual, such as a remote manual (like a database on a server). Yet even more especially, the invention provides an embodiment of the kit, wherein the manual includes instructions how to extract biological sample from a pregnant subject and/or how to use the analyzing unit and/or how to use the evaluation unit.

In an embodiment, the evaluation unit comprises a color scheme, wherein the analyzing unit is configured to provide a color reaction wherein the color is dependent upon the amount of ESM-1 in an extracted biological sample from a pregnant subject. Especially, the invention provides an embodiment of the kit comprising: (b) an evaluation unit comprising a data processor for determining whether a pregnant subject is likely to have or develop preeclampsia, eclampsia, and/or HELLP. Especially, the invention provides an embodiment of the kit comprising: (b) an evaluation unit comprising a data processor having implemented necessary algorithms for comparing the amount ESM-1 determined with reference values stored in a database in order to determine whether a pregnant subject is likely to have or develop the pregnancy related syndrome. In a specific embodiment of the kit, said kit comprises: (a) an analyzing unit comprising a detection agent for ESM-1 and one or more of sFlt1, VEGF, PlGF, HGF, sEndoglin, pp-13, PAPP-A and GDF-15. Hence, the invention may also provide an embodiment of the kit comprising and analyzing unit comprising a detection agent for ESM-1, and a detection agent for one or more of sFlt1, VEGF, PlGF, HGF, sEndoglin, pp-13, PAPP-A and GDF-15, which allows the determination of the amount of ESM-1, and one or more of sFlt1, VEGF, PlGF, HGF, sEndoglin, pp-13, PAPP-A and GDF-15. Yet even more especially, the invention provides an embodiment of the kit comprising an evaluation unit comprising a data processor having implemented necessary algorithms for comparing the amount ESM-1, and one or more of sFlt1, VEGF, PlGF, HGF, sEndoglin, pp-13, PAPP-A and GDF-15 determined with reference values stored in a database and calculate the ratio between the measured markers in order to determine whether a pregnant subject is likely to have or develop the pregnancy related syndrome. Alternative or additional to the above indicated secondary marker, also one or more or pikachurin and a hemopexin may be chosen. As indicated above, in an embodiment the analyzing unit may comprise an assay. Even more especially, in an embodiment the analyzing unit comprises an ELISA (Enzyme-linked immunosorbent assay) assay, a turbidimetric assay, radioimmunosorbent assay (RIST), radioimmunoassay (RIA), direct or indirect immunofluorescence, particle gel immuno assay (PaGIA), or a lateral flow assay.

In an embodiment (of the kit), the manual includes information to extract biological sample from a pregnant subject in any one week of gestation, especially between week 1 to 20 of gestation, even more especially between weeks 12 to 16.

Alternatively or additionally the invention provides an embodiment of the kit wherein the analyzing unit comprises a method to do a quantification of the RNA coding for ESM-1 in cells or cell-free RNA in the biological sample, especially a biological fluid, such as a body liquid.

Hence, the method, kit or device of the invention may be used for predicting a pregnant subject in any one of week 1 to 20 of gestation to develop after week 20 early onset severe preeclampsia, based on a measurement of the amount of ESM-1 in a biological fluid sample from the pregnant subject, wherein the biological fluid sample especially comprises a blood sample, a plasma sample, or a serum sample from said pregnant subject. This will especially be the case when the ESM-1 amount is substantially smaller than the ESM-1 amount in a sample (from a pregnant subject at the same time, especially in about the same week of gestation) of a healthy pregnant subject.

As used herein the term "ESM-1" or "endocan" has its general meaning in the art and refers to the endothelial cell specific molecule-1 that is a 50-kDa dermatan sulfate proteoglycan expressed by endothelial cells in lung and kidney, among other cell types, and can be detected in human blood. As used herein the term "blood sample" refers to a whole blood, serum, or plasma sample. Typically the blood sample is drawn/collected from a patient by a physician or nurse and processed in the laboratory of the hospital. The above indicated assays may also be used in peripheral hospitals or practices and could even be in general use by midwives. Once the blood sample from the patient is prepared, the concentration of ESM-1 may be measured by any known method in the art. It is for instance referred to WO2012098219. For example, the concentration of ESM-1 may be measured by using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radio immunoassays; Immuno electrophoresis; immune precipitation, high performance liquid chromatography (FIPLC), size exclusion chromatography, solid-phase affinity, etc. As also described in WO2012098219, such methods may comprise contacting the blood sample with a binding partner capable of selectively interacting with ESM-1 present in the blood sample. The binding partner may be generally an antibody that may be polyclonal or monoclonal, preferably monoclonal. Polyclonal antibodies directed against ESM-1 can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies against ESM-1 can be prepared and isolated using any technique that provides for the production of antibody molecules by continuous cell lines in culture. Techniques for production and isolation include but are not limited to the hybridoma technique originally described WO2012098219 and references cited therein. Antibodies useful in practicing the present invention also include anti-ESM-1 fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv (single chain Fv) expression libraries can be constructed to allow rapid identification of fragments having the desired specificity to ESM-1. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay. Anti-ESM-1 monoclonal antibodies are commercially available from, for instance, Lunginnov (Lille, France), see also WO2012098219.

In another embodiment, the binding partner may be an aptamer. Aptamers are a class of molecules that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in WO2012098219 and references cited therein. The random sequence library is obtainable by combinatorial chemical synthesis of DNA. In this library, each member is a linear oligomer, eventually chemically modified, of a unique sequence. Possible modifications, uses and advantages of this class of molecules have been indicated and described in WO2012098219, and references cited therein. The binding partners of the invention such as antibodies or aptamers, may be labelled with a detectable molecule or substance, such as a fluorescent molecule, a radioactive molecule or any others labels known in the art. Labels are known in the art that generally provide (either directly or indirectly) a signal.

Early onset severe PE especially belongs to the herein described pregnancy related syndromes (such as preeclampsia). As used herein, the term "labeled", with regard to the antibody, is intended to encompass direct labeling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labeling of the probe or antibody by reactivity with a detectable substance. An antibody or aptamer of the invention may be labeled with a radioactive molecule by any method known in the art. For example radioactive molecules include but are not limited radioactive atom for scintigraphic studies such as 1123, 1124, Inl l l, Rel 86, Rel88. As also indicated in WO2012098219, the aforementioned assays generally involve the bounding of the binding partner (i.e. Antibody or aptamer) in a solid support. Solid supports which can be used in the practice of the invention include substrates such as nitrocellulose (e. g., in membrane or micro titer well form); polyvinylchloride (e. g., sheets or micro titer wells); polystyrene latex (e.g., beads or micro titer plates); polyvinylidine fluoride; diazotized paper; nylon membranes; activated beads, magnetically responsive beads, and the like.

More particularly, an ELISA method can be used, wherein the wells of a micro titer plate are coated with a set of antibodies against ESM-1. A blood sample containing or suspected of containing ESM-1 is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art. Typically an ELISA kit is commercially available from Lunginnov (Lille, France) or other sources, such as described in WO2012098219 and references cited therein. Measuring the concentration of ESM-1 (with or without immunoassay-based methods) may also include separation of the proteins: centrifugation based on the protein's molecular weight; electrophoresis based on mass and charge; HPLC based on hydrophobicity; size exclusion chromatography based on size; and solid-phase affinity based on the protein's affinity for the particular solid-phase that is use. Once separated, ESM-1 may be identified based on the known "separation profile" e. g., retention time, for that protein and measured using standard techniques. Alternatively, the separated proteins may be detected and measured by, for example, a mass spectrometer. Hence, presence and quantification of the analyte can be shown and done by using generally accepted techniques like, for instance, the enzyme linked immuno sorbent assay (ELISA), either in sandwich or competitive set up, a lateral flow method, a magnetic or fluorescent particle (bead) based assay and western blot, etc.

In an embodiment the amount of sFlt1, VEGF, PlGF, HGF, sEndoglin, pp-13, PAPP-A or GDF-15, or a combination of these markers, is determined in a sample in addition to the amount of ESM-1 and wherein sFlt1, VEGF, PlGF, HGF, sEndoglin, pp-13, PAPP-A or GDF-15, or combination of these markers, are compared to a reference amount of ESM-1, sFlt1, VEGF, PlGF, HGF, sEndoglin, pp-13, PAPP-A or GDF-15 for diagnosing the risk of experiencing preeclampsia, eclampsia, or HELLP. In an embodiment the ratio between ESM-1 and any of said markers or a combination of said markers in a sample is used to determine the predisposition of a pregnant subject for the development of preeclampsia, eclampsia, or HELLP, or not. In an embodiment said sample is a blood, plasma, serum, urine or saliva from the subject. In an example said sample is derived from a subject being in any one week of gestation between week 1 to 20 of gestation. In an example said sample is derived from a subject being in any one week of gestation between week 20 to 36 of gestation. In an embodiment said measuring levels is done on two or more occasions and a change in said levels is a diagnostic value for the development of preeclampsia, eclampsia, or HELLP, or not. In an embodiment said measuring is done using an immunological assay. In a further aspect, the invention provides a device to determine the predisposition of a pregnant subject to develop preeclampsia, eclampsia, or HELLP, or not, said device comprising: (a) an analyzing unit comprising a detection agent for ESM-1, which allows the determination of the amount of ESM-1; (b) an evaluation unit comprising a data processor having implemented necessary algorithms for comparing the amount ESM-1 determined with reference values stored in a database in order to determine the predisposition of a pregnant woman to develop preeclampsia, eclampsia, or HELLP, or not.

In an embodiment, the invention further provides a device to determine the predisposition of a pregnant subject to develop preeclampsia, eclampsia, or HELLP, or not, said device comprising: (a) an analyzing unit comprising a detection agent for ESM-1, sFlt1, VEGF, PlGF, HGF, sEndoglin, pp-13, PAPP-A or GDF-15, which allows the determination of the amount of ESM-1, sFlt1, VEGF, PlGF, HGF, sEndoglin, pp-13, PAPP-A or GDF-15; (and/or) (b) an evaluation unit comprising a data processor having implemented necessary algorithms for comparing the amount ESM-1, sFlt1, VEGF, PlGF, HGF, sEndoglin, pp-13, PAPP-A or GDF-15 determined with reference values stored in a database and calculate the ratio between the measured markers in order to determine the predisposition of a pregnant woman to develop preeclampsia, eclampsia, or HELLP, or not. In an embodiment, the invention further provides a device, wherein said evaluation unit is also capable of giving therapeutic recommendations. In yet a further aspect, the invention also provides a kit to determine the predisposition of a pregnant subject to develop preeclampsia, eclampsia, or HELLP, or not, said kit comprising, at least the detection agent for ESM-1 and preferably, standards which reflect the reference amounts as derived from a pregnant subject or a group thereof known not to suffer or having predisposition to develop preeclampsia, eclampsia, or HELLP.

The amount of ESM-1 in the biological (fluid) sample of the pregnant subject is especially compared with a reference value derived from a biological (fluid) sample of pregnant subjects having a healthy pregnancy, and wherein the biological samples (i.e. reference samples and the biological sample of the pregnant subject) have been produced in the same way (same type of sample) and in the same week.

The term "substantially" herein, such in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species". Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. However, the terms first and second, etc., may also indicate a relation in time. For instance, a first sample may be extracted earlier in time than a second sample. Especially, this applies to the terms "first occasion" and "second occasion". Based on the measured values, a diagnosis may be made.

The devices herein may amongst others described during operation. As will be clear to the person skilled in the art, the invention is not limited to methods of operation or devices in operation. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage. The invention further applies to a device comprising one or more of the characterizing features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

### DESCRIPTION OF THE DRAWINGS

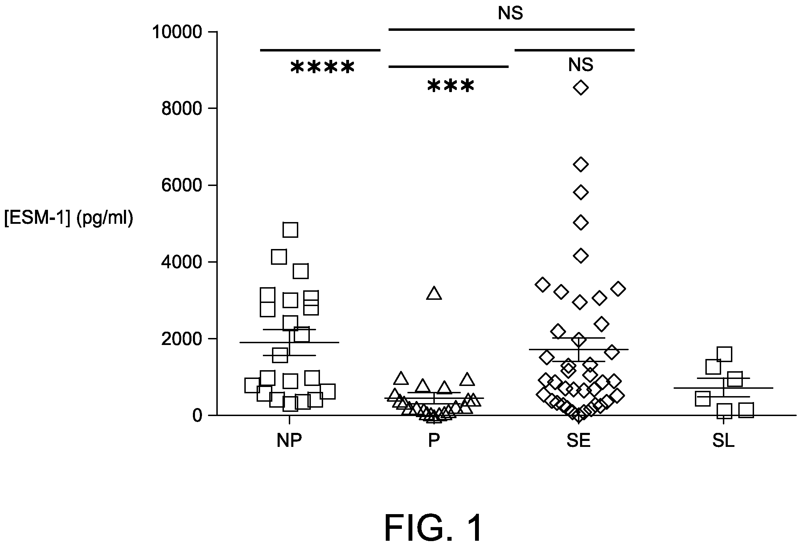

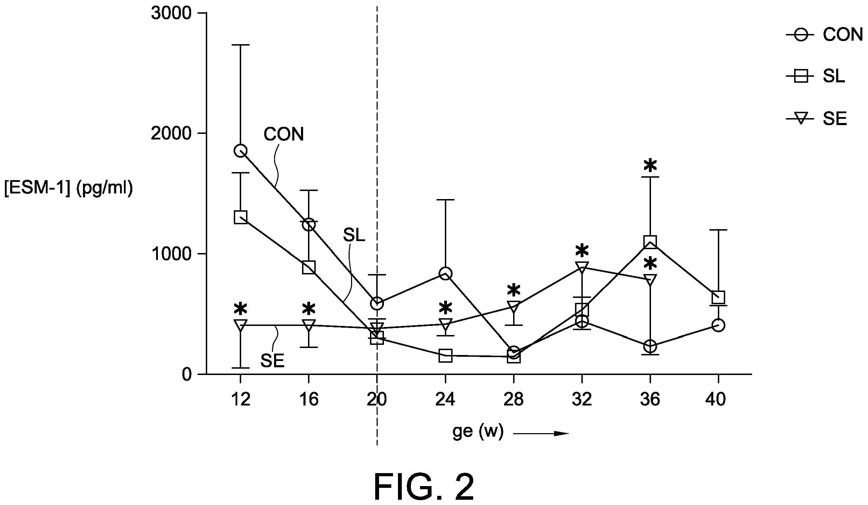

Figure 1: ESM-1 concentration in plasma during the last trimester of healthy pregnant (P) women (n=32), severe early-onset preeclampsia (SE) women (n=52), and severe late-onset (SL) women (n=8) preeclampsia and from non pregnant (NP) women (n=25). Significance calculate by Mann Whitney test: **** = p<0.0001, *** = p<0.001, and NS = not significant; and Figure 2. ESM-1 concentrations in plasma during the pregnancy (week 12 until birth) of healthy pregnant (CON; n=23), severe early-onset preeclampsia (SE; n=11) and severe late-onset preeclampsia (SL; n=7). Significance between healthy and preeclamptic pregnancies is calculated by Mann Whitney test: * = p<0.05. The gestation time is indicated with "ge" in weeks (w).

### DETAILED DESCRIPTION OF THE EMBODIMENTS

Since ESM-1 plays a role in the regulation of angiogenesis we hypothesized that the expression of ESM-1 during pregnancy would be different between the healthy and the two forms, early and late, of preeclampsia pregnancies. A total of 290 EDTA plasma samples from 23 healthy and GA matched control (CON), 11 severe early-onset PE (SE) and 7 severe late onset PE (SL) pregnancies was collected at regular intervals between week 8 and birth. In these plasma samples ESM-1 was measured by ELISA. For reasons of convenience the mean of the values was determined of 4 week periods; 9 through 12 are expressed as week 12, weeks 13 through 16 are expressed as week 16, etc. During the period between GA week 24 and birth ESM-1 concentrations were significantly increased in both early and late preeclampsia when compared to controls during the same period. Surprisingly the concentration of ESM-1 also differed between the three groups between weeks 12 and 16. The ESM-1 concentration of the healthy pregnancies (mean of 1857 pg/ml) and those that develop severe late-onset PE (mean of 1298 pg/ml) are comparable, but in those pregnancies that develop severe early-onset PE the concentration (mean of 410 pg/ml) is significantly lower (figure 1). These results indicate that ESM-1 is a prognostic marker which can determine the risk of women to develop severe early-onset PE already as early as 12 to 16 weeks of gestation (figure 2).

Patients with early-onset preeclampsia and healthy pregnant controls were recruited from the antenatal ward and the non-pregnant women were recruited from hospital staff and students. Exclusion criteria for all groups were pre-existing hypertension, diabetes mellitus, vasculitis, renal disease, autoimmune disease, malignancies or women who had recent trauma or surgery. Preeclampsia was defined according to the standards of the International Society for the Study of Hypertension in Pregnancy (ISSHP): diastolic blood pressure of 90 mmHg or more on two or more consecutive occasions more than 4 hrs. apart and proteinuria of more than 300 mg/24 hours. Early-onset preeclamptic women have been included, defined as the onset of preeclampsia before 34 weeks. Blood samples were taken from non-pregnant women and from patients with preeclampsia and healthy pregnant women between week 12 of pregnancy and birth.

Maternal blood samples of both pregnant and preeclamptic women were collected during routine blood sampling. Blood samples were drawn into 10 mL tubes containing EDTA (Venoject, Terumo Europe NV, Leuven, Belgium). From these samples the plasma was collected and frozen (-80°C) in aliquots until the assays could be performed simultaneously. The plasma levels of all analytes were determined using commercially available reagents. These reagents were either present in sets or kits to perform sandwich ELISA assays. The sFlt, sEng, PlGF and HGF were measured by Quantikine ELISAs (R&D Systems Inc., Minneapolis, US). The ESM-1 was measured using the antibody combination and standard from Lunginnov (Lille, France).

In short; a first mono- or polyclonal antibody specific for the analyte was immobilized in an ELISA micro plate. After overnight incubation the plate was washed and blocked with phosphate buffer saline (PBS) containing 0.1% bovine serum albumin. After subsequent incubation the plate was washed with washing buffer containing 0.1% Tween-20. From a stock solution containing a known concentration of the analyte a standard curve is prepared by 1 to 1 dilution. 100 µL aliquots of the standard curve or the plasma sample were incubated in the different wells of the ELISA plate. After incubation the plate has been washed again and followed by the incubation with a second mono- or polyclonal antibody specific for the analyte and conjugated with biotin. Before incubating the assay with streptavidin-poly-HRPO the plate was washed again. After the incubation with streptavidin-poly-HRPO the assay is in incubated with 3,3',5,5'-Tetramethylbenzidine (TMB) Substrate solution. The HRPO enzyme present in the well will turn the colorless solution blue proportion to the analyte present. The color development was stopped and the intensity of the color was measured. Further details can be found in the respective package inserts. As indicated above, a total of 290 EDTA plasma samples from 23 healthy and GA matched control (CON), 11 severe early-onset PE (SE) and 7 severe late-onset PE (SL) pregnancies was collected at regular intervals between week 8 and birth. In these plasma samples ESM-1 was measured by ELISA. During the period between GA week 24 and birth ESM-1 concentrations were significantly increased in both early and late preeclampsia when compared to controls during the same period. Surprisingly, the concentration of ESM-1 also differed between the three groups between weeks 12 and 16. The ESM-1 concentration of the healthy pregnancies (mean of 1857 pg/ml) and those that develop severe late-onset PE (mean of 1298 pg/ml) are comparable, but in those pregnancies that develop severe early-onset PE the concentration (mean of 410 pg/ml) is significantly lower (figure 1). These results indicate that ESM-1 is a prognostic marker which can determine the risk of women to develop severe early-onset PE already as early as 12 to 16 weeks of gestation (figure 2). Based on the measurement of the ESM-1 concentration in blood the risk of pregnant woman to develop preeclampsia later in her pregnancy can be determined. This risk is higher when the ESM-1 concentration is lower than that of women that will not develop preeclampsia or late-onset preeclampsia.

In an aspect, the invention provides a method for identifying a pregnant subject that is likely to have or develop preeclampsia, eclampsia, and/or Hemolysis Elevated Liver enzymes and Low Platelets (HELLP), comprising the steps of: (a) extracting a biological sample from a subject; (b) using an assay specific to ESM-1, measuring the amount of ESM-1 in the biological sample; and (c) comparing said amount of ESM-1 to a reference value; and (d) identifying the subject as being likely to have or develop preeclampsia, eclampsia, and/or HELLP based on a comparison of the amount of ESM-1 to a reference value.

In yet a further aspect, the invention provides a method for identifying a pregnant subject that is likely to have or develop preeclampsia, eclampsia, Hemolysis Elevated Liver enzymes and/or Low Platelets (HELLP), comprising the steps of: (a) extracting a biological sample from a subject; (b) using an assay specific to ESM-1, measuring the amount of ESM-1 in the biological sample; and (c) comparing said amount of ESM-1 to a reference value; and (d) identifying the subject as being likely to have or develop preeclampsia, eclampsia, and/or HELLP when said amount of ESM-1 is greater than the reference value.

In yet a further aspect, the invention provides a method for identifying a pregnant subject that is likely to have or develop preeclampsia, eclampsia, and/or HELLP, comprising the steps of: (a) extracting a biological sample from a subject; (b) using an assay specific to ESM-1, measuring the amount of ESM-1 in the biological sample; (c) comparing said amount of ESM-1 to an ESM-1 reference value; and (d) selecting one or more secondary markers from the group consisting of soluble Fms-like tyrosine kinase-1 (sFlt1), Vascular Endothelial Growth Factor (VEGF), Placental Growth Factor (PlGF), Hepatocyte Growth Factor (HGF), soluble Endoglin, placental protein 13 (pp-13), Pregnancy- associated Plasma Protein A (PAPP-A) and Growth Differentiation Factor 15 (GDF-15); (e) using assays specific for each of the one or more secondary markers, measuring the amounts of each of the one or more secondary markers in the biological sample; (f) for each of said one or secondary markers, selecting a secondary marker reference value, and comparing said amount of said secondary marker to the secondary marker reference value; and (g) identifying the subject as being likely to have or develop preeclampsia, eclampsia, HELLP and/or IUGR based on a comparison of the amount ESM-1 with a reference value, and based on a comparison of the amount of said one or more secondary markers with the corresponding secondary marker reference value.

In yet a further aspect, the invention provides a method for identifying a pregnant subject that is likely to have or develop preeclampsia, eclampsia, and/or HELLP, comprising the steps of: (a) extracting a biological sample from a subject; (b) using an assay specific to ESM-1, measuring the amount of ESM-1 in the biological sample; (c) comparing said amount of ESM-1 to an ESM-1 reference value; and (d) selecting one or more secondary markers from the group consisting of soluble Fms-like tyrosine kinase-1 (sFlt1), Vascular Endothelial Growth Factor (VEGF), Placental Growth Factor (PlGF), Hepatocyte Growth Factor (HGF), soluble Endoglin, placental protein 13 (pp-13), Pregnancy- associated Plasma Protein A (PAPP-A) and Growth Differentiation Factor 15 (GDF-15); (e) using assays specific for each of the one or more secondary markers, measuring the amounts of each of the one or more secondary markers in the biological sample; (f) for each of said one or secondary markers, selecting a secondary marker reference value, and comparing said amount of said secondary marker to the secondary marker reference value; and (g) identifying the subject as being likely to have or develop preeclampsia, eclampsia, and/or HELLP when said amount of ESM-1 is greater than the ESM-1 reference value, and when said amount of each of said one or more secondary markers is greater than its corresponding secondary marker reference value.

In yet a further aspect, the invention provides a method for identifying a pregnant subject that is likely to have or develop preeclampsia, eclampsia, and/or HELLP, comprising the steps of: (a) extracting a biological sample from a subject; (b) using an assay specific to ESM-1, measuring the amount of ESM-1 in the biological sample; (c) selecting one or more secondary markers from the group consisting of sFlt1, VEGF, PlGF, HGF, sEndoglin, pp-13, PAPP-A and GDF-15; (d) using assays specific for each of the one or more secondary markers, measuring the amount of each of the one or more secondary markers in the biological sample; (e) for each of the secondary markers: (i) determining the ratio of the amount of ESM-1 to the amount of the secondary marker; and, (ii)selecting a ratio reference value; (iii) comparing the ratio with the ratio reference value; and (f) identifying the subject as being likely to have or develop preeclampsia, eclampsia, and/or HELLP, especially based on a comparison with the ratio of a reference, such as when for each of the secondary markers, said ratio is greater than the ratio reference value.

In yet a further aspect, the invention provides a method for identifying a pregnant subject that is likely to have or develop preeclampsia, eclampsia, and/or HELLP, comprising the steps of: (a) extracting a first biological sample from a subject on a first occasion; (b) extracting a second biological sample from a subject on a second occasion; (c) using an assay specific to ESM-1, measuring the amount of ESM-1 in the first biological sample, constituting a first amount; (d) using an assay specific to ESM-1, measuring the amount of ESM-1 in the second biological sample, constituting a second amount; and (e) identifying the subject as being likely to have or develop preeclampsia, eclampsia, and/or HELLP especially based on a comparison of the first amount and the second amount, such as when the second amount is greater than the first amount by a pre-defined reference value. In yet a further aspect, the invention provides a method for identifying a pregnant subject that is likely to have or develop preeclampsia, eclampsia, and/or HELLP, comprising the steps of (a) extracting a first biological sample from a subject; (b) extracting a second biological sample from a subject; (c) using an assay specific to ESM-1, measuring the amount of ESM-1 in the first biological sample, constituting a first amount of ESM-1; (d) using an assay specific to ESM-1, measuring the amount of ESM-1 in the second biological sample, constituting a second amount of ESM-1; (e) selecting one or more secondary markers from the group consisting of sFlt1, VEGF, PlGF, HGF, sEndoglin, pp-13, PAPP-A and GDF-15; (f) using assays specific for each of the one or more secondary markers, measuring the amounts of each of the one or more secondary markers in the first biological sample, constituting a set of first amounts of the secondary markers; (g) using assays specific for each of the one or more secondary markers, measuring the amounts of each of the one or more secondary markers in the second biological sample, constituting a set of second amounts of the secondary markers; (h) identifying the subject as being likely to have or develop preeclampsia, eclampsia, and/or HELLP especially based (i) on a comparison of the first amount of ESM-1 and the second amount of ESM-1 and/or based (ii) on a comparison of the second amount of the secondary marker and the first amount, such as when the second amount of ESM-1 is greater than the first amount of ESM-1 by a pre-defined ESM-1 reference value, and for each of the secondary markers, the second amount of the secondary marker is greater than the first amount of the secondary marker by a pre-defined reference value for that secondary marker.

In yet a further aspect, the invention provides a method for identifying a pregnant subject that is likely to have or develop preeclampsia, eclampsia, and/or HELLP, comprising the steps of: (a) extracting a first biological sample from a subject; (b) extracting a second biological sample from a subject; (c) using an assay specific to ESM-1, measuring the amount of ESM-1 in the first biological sample, constituting a first amount of ESM-1; (d) using an assay specific to ESM-1, measuring the amount of ESM-1 in the second biological sample, constituting a second amount of ESM-1; (e) selecting one or more secondary markers from the group consisting of sFlt1, VEGF, PlGF, HGF, sEndoglin, pp-13, PAPP-A and GDF-15; (f) using assays specific for each of the one or more secondary markers, measuring the amounts of each of the one or more secondary markers in the first biological sample, constituting a set of first amounts of the secondary markers; (g) using assays specific for each of the one or more secondary markers, measuring the amounts of each of the one or more secondary markers in the second biological sample, constituting a set of second amounts of the secondary markers; (h) for each of the secondary markers: (1) determining the ratio of the first amount of ESM-1 to the first amount of the secondary marker, constituting a first ratio; (2) determining the ratio of the second amount of ESM-1 to the second amount of the secondary marker, constituting a second ratio; (3) comparing the first ratio to the second ratio; and (i) identifying the subject as being likely to have or develop preeclampsia, eclampsia, and/or HELLP, especially when for each of the secondary markers, the second ratio is greater than the first ratio by a pre-defined reference value.

In an embodiment, he method further comprises the step of uterine artery Doppler screening. In an embodiment, said step(s) of measuring are done using one or more immunological assays, especially wherein the one or more immunological assay comprise an ELISA (Enzyme-linked immunosorbent assay) assay, a turbidimetric assay, radioimmunosorbent assay (RIST), radioimmunoassay (RIA), direct or indirect immunofluorescence, particle gel immuno assay (PaGIA), mass spectrometry (MS) or lateral flow assay. In an embodiment, said immunological assay(s) are an ELISA. In an embodiment, said immunological assay(s) are a lateral flow assay. In an embodiment, said step(s) of measuring are done using one or more enzymatic colorimetric assays. In an embodiment, said step(s) of measuring are done using mass spectrometry (MS). In an embodiment, said amount of ESM-1 is the level of free ESM-1, bound ESM-1, a metabolite of ESM-1 or total ESM-1.

In yet a further aspect, the invention provides a device for identifying a pregnant subject being in any one week 9-16 of gestation that is likely to have or develop preeclampsia, eclampsia, and HELLP, said device comprising: (a) An analyzing unit comprising a detection agent for ESM-1, for instance being an antibody, a (recombinant) receptor for ESM-1 or an aptamer, which allows the determination of the amount of ESM-1; (b) An evaluation unit comprising a data processor having implemented necessary algorithms for comparing the amount ESM-1 determined with reference values stored in a database in order to determine whether a pregnant subject is likely to have or develop preeclampsia, eclampsia, and/or HELLP. In yet a further aspect, the invention provides a device for identifying a pregnant subject that is likely to have or develop preeclampsia, eclampsia, and/or HELLP, said device comprising: (a) An analyzing unit comprising a detection agent for ESM-1, sFlt1, VEGF, PlGF, HGF, sEndoglin, pp-13, PAPP-A or GDF-15, which allows the determination of the amount of ESM-1, sFlt1, VEGF, PlGF, HGF, sEndoglin, pp-13, PAPP-A or GDF-15; (b) An evaluation unit comprising a data processor having implemented necessary algorithms for comparing the amount ESM-1, sFlt1, VEGF, PlGF, HGF, sEndoglin, pp-13, PAPP-A or GDF-15 determined with reference values stored in a database and calculate the ratio between the measured markers in order to determine whether a pregnant subject is likely to have or develop preeclampsia, eclampsia, and/or HELLP. In an embodiment, said evaluation unit is also capable of giving therapeutic recommendations. In yet a further aspect, the invention provides a use of an evaluation of the amount of ESM-1 in an extracted biological sample from a pregnant subject for identifying whether the pregnant subject is likely to have or develop preeclampsia, eclampsia, and/or HELLP.

In yet a further embodiment, the invention provides the in vitro use of an extracted biological sample from a pregnant subject for identifying whether the pregnant subject is likely to have or develop preeclampsia, eclampsia, and/or HELLP, the use comprising: (a) using an assay specific to ESM-1, measuring the amount of ESM-1 in the biological sample; (b) comparing said amount of ESM-1 to a reference value; and (c) identifying the subject as being likely to have or develop preeclampsia, eclampsia, HELLP and/or IUGR. In an embodiment, the use (further) comprises identifying the subject as being likely to have or develop preeclampsia, eclampsia, and/or HELLP based on a comparison of the amount of ESM-1 to a reference value. In an example, the use (further) comprises identifying the subject as being likely to have or develop preeclampsia, eclampsia, HELLP and/or IUGR when said amount of ESM-1 is greater than the reference value, especially when the biological sample is from a pregnant subject in any one week of gestation between week 26 or later of gestation.

In yet a further embodiment, the invention provides the in vitro use of an extracted biological sample from a pregnant subject for identifying whether the pregnant subject is likely to have or develop preeclampsia, eclampsia, and/or HELLP, the use comprising: (a) using an assay specific to ESM-1, measuring the amount of ESM-1 in the biological sample; (b) comparing said amount of ESM-1 to an ESM-1 reference value; and (c) selecting one or more secondary markers from the group consisting of sFlt1, VEGF, PlGF, HGF, sEndoglin, pp-13, PAPP-A and GDF-15; (d) using assays specific for each of the one or more secondary markers, measuring the amounts of each of the one or more secondary markers in the biological sample; (e) for each of said one or secondary markers, selecting a secondary marker reference value, and comparing said amount of said secondary marker to the secondary marker reference value; and (f) identifying the subject as being likely to have or develop preeclampsia, eclampsia, and/or HELLP.

In an embodiment, the use (further) comprises identifying the subject as being likely to have or develop preeclampsia, eclampsia, and/or HELLP based on a comparison of the amount ESM-1 with a reference value, and based on a comparison of the amount of said one or more secondary markers with the corresponding secondary marker reference value. In an example, the use (further) comprises identifying the subject as being likely to have or develop preeclampsia, eclampsia, HELLP and/or IUGR when said amount of ESM-1 is greater than the ESM-1 reference value, especially when the biological sample is from a pregnant subject in any one week of gestation between week 26 or later of gestation. In an embodiment, the use (further) comprises identifying the subject as being likely to have or develop preeclampsia, eclampsia, and/or HELLP when said amount of each of said one or more secondary markers is greater than its corresponding secondary marker reference value.

In yet a further aspect, the invention provides a in vitro use of an extracted biological sample from a pregnant subject for identifying a pregnant subject that is likely to have or develop preeclampsia, eclampsia, and/or HELLP, comprising the steps of: (a) using an assay specific to ESM-1, measuring the amount of ESM-1 in the biological sample; (b) selecting one or more secondary markers from the group consisting of sFlt1, VEGF, PlGF, HGF, sEndoglin, pp-13, PAPP-A and GDF-15; (c) using assays specific for each of the one or more secondary markers, measuring the amount of each of the one or more secondary markers in the biological sample; (d) for each of the secondary markers: (i) determining the ratio of the amount of ESM-1 to the amount of the secondary marker; and, (ii) selecting a ratio reference value; (iii) comparing the ratio with the ratio reference value; and (e) identifying the subject as being likely to have or develop preeclampsia, eclampsia, and/or HELLP.

In an embodiment, the use (further) comprises identifying the subject as being likely to have or develop preeclampsia, eclampsia, and/or HELLP based on a comparison for each of the secondary markers with the ratio reference value. In an embodiment, the use (further) comprises identifying the subject as being likely to have or develop preeclampsia, eclampsia, and/or HELLP when for each of the secondary markers, said ratio is greater than the ratio reference value. In an embodiment said sample is derived from a subject being in any one week of gestation between week 1 to 20 of gestation, especially between weeks 12 to 16, and identifying the subject as being likely to have or develop preeclampsia, eclampsia, and/or HELLP when said amount of ESM-1 is smaller than the ESM-1 reference value.

In yet a further aspect, the invention provides a in vitro use of extracted biological samples from a pregnant subject for identifying a pregnant subject that is likely to have or develop preeclampsia, eclampsia, and/or HELLP, using a first biological sample from a subject extracted on a first occasion and using a second biological sample from a subject extracted on a second occasion, comprising the steps of: (a) using an assay specific to ESM-1, measuring the amount of ESM-1 in the first biological sample, constituting a first amount; (b) using an assay specific to ESM-1, measuring the amount of ESM-1 in the second biological sample, constituting a second amount; and (c) identifying the subject as being likely to have or develop preeclampsia, eclampsia, and/or HELLP. In an embodiment, the use (further) comprises identifying the subject as being likely to have or develop preeclampsia, eclampsia, and/or HELLP based on a comparison of the second amount and the first amount. In an example, the use (further) comprises identifying the subject as being likely to have or develop preeclampsia, eclampsia, HELLP and/or IUGR when the second amount is greater than the first amount by a pre-defined reference value, especially when the biological sample is from a pregnant subject in any one week of gestation between week 26 or later of gestation. In an embodiment, the use (further) comprises identifying the subject as being likely to have or develop preeclampsia, eclampsia, and/or HELLP when the second amount is smaller than the first amount by a pre-defined reference value when the biological sample is from a pregnant subject in any one week of gestation before week 26, especially before week 20, even more especially in any week of gestation between week 12 and 16 of gestation.

In yet a further aspect, the invention provides a in vitro use of extracted biological samples from a pregnant subject for identifying a pregnant subject that is likely to have or develop preeclampsia, eclampsia, and/or HELLP, using a first biological sample from a subject extracted on a first occasion and using a second biological sample from a subject extracted on a second occasion, comprising the steps of: (a) using an assay specific to ESM-1, measuring the amount of ESM-1 in the first biological sample, constituting a first amount of ESM-1; (b) using an assay specific to ESM-1, measuring the amount of ESM-1 in the second biological sample, constituting a second amount of ESM-1; (c) selecting one or more secondary markers from the group consisting of sFlt1, VEGF, PlGF, HGF, sEndoglin, pp-13, PAPP-A and GDF-15; (d) using assays specific for each of the one or more secondary markers, measuring the amounts of each of the one or more secondary markers in the first biological sample, constituting a set of first amounts of the secondary markers; (e) using assays specific for each of the one or more secondary markers, measuring the amounts of each of the one or more secondary markers in the second biological sample, constituting a set of second amounts of the secondary markers; (f) identifying the subject as being likely to have or develop preeclampsia, eclampsia, and/or HELLP.

In an example, the use (further) comprises identifying the subject as being likely to have or develop preeclampsia, eclampsia, HELLP and/or IUGR when the second amount is greater than the first amount by a pre-defined reference value, especially when the biological sample is from a pregnant subject in any one week of gestation between week 26 or later of gestation. In an embodiment, the use (further) comprises identifying the subject as being likely to have or develop preeclampsia, eclampsia, and/or HELLP when the second amount is smaller than the first amount by a pre-defined reference value when the biological sample is from a pregnant subject in any one week of gestation before week 26, especially before week 20, even more especially in any week of gestation between week 12 and 16 of gestation.

In an embodiment, the use (further) comprises identifying the subject as being likely to have or develop preeclampsia, eclampsia, and/or HELLP based on a comparison of each of said one or more secondary markers with its corresponding secondary marker reference value. In an example, the use (further) comprises identifying the subject as being likely to have or develop preeclampsia, eclampsia, HELLP and/or IUGR when said amount of each of said one or more secondary markers is (also) greater than its corresponding secondary marker reference value.

In yet a further aspect, the invention provides a in vitro use of extracted biological samples from a pregnant subject for identifying a pregnant subject that is likely to have or develop preeclampsia, eclampsia, and/or HELLP, using a first biological sample from a subject extracted on a first occasion and using a second biological sample from a subject extracted on a second occasion, comprising the steps of: (a) using an assay specific to ESM-1, measuring the amount of ESM-1 in the first biological sample, constituting a first amount of ESM-1; (b) using an assay specific to ESM-1, measuring the amount of ESM-1 in the second biological sample, constituting a second amount of ESM-1; (c) selecting one or more secondary markers from the group consisting of sFlt1, VEGF, PlGF, HGF, sEndoglin, pp-13, PAPP-A and GDF-15; (d) using assays specific for each of the one or more secondary markers, measuring the amounts of each of the one or more secondary markers in the first biological sample, constituting a set of first amounts of the secondary markers; (e) using assays specific for each of the one or more secondary markers, measuring the amounts of each of the one or more secondary markers in the second biological sample, constituting a set of second amounts of the secondary markers; (f) for each of the secondary markers: (i) determining the ratio of the first amount of ESM-1 to the first amount of the secondary marker, constituting a first ratio; (ii) determining the ratio of the second amount of ESM-1 to the second amount of the secondary marker, constituting a second ratio; (iii) comparing the first ratio to the second ratio; and (g) identifying the subject as being likely to have or develop preeclampsia, eclampsia, and/or HELLP.

In an embodiment, the use (further) comprises identifying the subject as being likely to have or develop preeclampsia, eclampsia, and/or HELLP based on a comparison of the ratio each of the secondary markers with a pre-defined reference value. In an embodiment, the use (further) comprises identifying the subject as being likely to have or develop preeclampsia, eclampsia, and/or HELLP when for each of the secondary markers, the second ratio is greater than the first ratio by a pre-defined reference value. In an embodiment, said step(s) of measuring are done using one or more immunological assays. In an embodiment, said immunological assay(s) are an ELISA. In an embodiment, said immunological assay(s) are a lateral flow assay. In an embodiment, said step(s) of measuring are done using one or more of an enzymatic or a colorimetric or a luminescent assay. In an embodiment, said step(s) of measuring are done using mass spectrometry (MS). In an embodiment, said amount of ESM-1 is the level of free ESM-1, bound ESM-1, a metabolite of ESM-1 or total ESM-1.

In an embodiment, one or more of said first biological sample and said second biological sample, especially both the first biological sample and the second biological sample, is a blood, plasma, serum, urine or saliva from the subject. In an embodiment, one or more of said first biological sample and said second biological sample, especially both the first biological sample and the second biological sample, is derived from a subject being in any one week of gestation between week 1 to 20 of gestation, especially between weeks 12 to 16. In a further example, one or more of said first biological sample and said second biological sample, especially both the first biological sample and the second biological sample, is derived from a subject being in any one week of gestation between week 20 to 36 of gestation.

In yet a further aspect, the invention provides a kit for identifying a pregnant subject that is likely to have or develop preeclampsia, eclampsia, and/or HELLP, said kit comprising: (a) An analyzing unit comprising a detection agent for ESM-1; (b) an evaluation unit for determining whether a pregnant subject is likely to have or develop preeclampsia, eclampsia, and/or HELLP, based on a result provided by the analyzing unit. In an embodiment, the kit may further comprise a manual or a reference to a (remote) manual. In an embodiment, the manual includes instructions how to extract biological sample from a pregnant subject and/or how to use the analyzing unit and/or how to use the evaluation unit. In an embodiment, the evaluation unit comprises a color scheme, wherein the analyzing unit is configured to provide a color reaction wherein the color is dependent upon the amount of ESM-1 in an extracted biological sample from a pregnant subject. In an embodiment, the kit (further) comprises: (a) An evaluation unit comprising a data processor for determining whether a pregnant subject is likely to have or develop preeclampsia, eclampsia, and/or HELLP. In an embodiment, the kit (further) comprises: (b) An evaluation unit comprising a data processor having implemented necessary algorithms for comparing the amount ESM-1 determined with reference values stored in a database in order to determine whether a pregnant subject is likely to have or develop preeclampsia, eclampsia, and/or HELLP.

In yet a further aspect, the invention provides a kit for identifying a pregnant subject that is likely to have or develop preeclampsia, eclampsia, and/or HELLP, said kit comprising: (a) An analyzing unit comprising a detection agent for ESM-1, sFlt1, VEGF, PlGF, HGF, sEndoglin, pp-13, PAPP-A or GDF-15. In yet a further aspect, the invention provides a kit as described herein for identifying a pregnant subject that is likely to have or develop preeclampsia, eclampsia, and/or HELLP, said kit comprising: (a) An analyzing unit comprising a detection agent for ESM-1, sFlt1, VEGF, PlGF, HGF, sEndoglin, pp-13, PAPP-A or GDF-15, which allows the determination of the amount of ESM-1, sFlt1, VEGF, PlGF, HGF, sEndoglin, pp-13, PAPP-A or GDF-15.

In yet a further aspect, the invention provides a kit as described herein, said kit (further) comprising: (b) An evaluation unit comprising a data processor having implemented necessary algorithms for comparing the amount ESM-1, sFlt1, VEGF, PlGF, HGF, sEndoglin, pp-13, PAPP-A or GDF-15 determined with reference values stored in a database and calculate the ratio between the measured markers in order to determine whether a pregnant subject is likely to have or develop preeclampsia, eclampsia, HELLP and/or IUGR. In an embodiment, said evaluation unit is also capable of giving therapeutic recommendations. In an embodiment, the analyzing unit comprises an assay. In an embodiment, the analyzing unit comprises an ELISA (Enzyme-linked immunosorbent assay) assay, a turbidimetric assay, radioimmunosorbent assay (RIST), radioimmunoassay (RIA), direct or indirect immunofluorescence, particle gel immuno assay (PaGIA), mass spectrometry (MS) or lateral flow assay. In an embodiment, the manual includes information to extract biological sample from a pregnant subject in any one week of gestation, especially between week 1 to 20 of gestation, even more especially between weeks 12 to 16. The method and use, as well as the application of the kit may include taking a plurality of samples over a period of time and determining with the ESM-1 specific assay the amount(s) of ESM-1 or derivative values thereof like ratio's of values. Alternative or additionally, the quantification is an indirect quantification, for instance by a color reaction that may be dependent upon the concentration (amount). Based on the color, a prediction may be made about a pregnant subject whether she is likely to have or develop preeclampsia, eclampsia, Hemolysis Elevated Liver enzymes and Low Platelets (HELLP) and/or Intra Uterine Growth Restriction (IUGR).

Additionally or alternatively, reference values may be determined or provided, for instance in a manual (on the internet) based upon one can determine whether the pregnant subject has or is likely to develop one of the above-mentioned syndromes. When comparing the value obtained of the subject with a reference value, or a plurality of reference values when more than one parameter is determined, the status of likely status to be can be predicted. Hence, the manual and/or reference data can be remote, such as on the internet. The user may derive the date from the internet.

Measuring can be done using a method to quantify the amount of protein via the amount of RNA coding for the said protein. Hence, in an embodiment said step(s) of measuring are done using a method to quantify the amount of protein via the amount of RNA coding for the said protein. Further, additionally or alternatively said quantification can be done via RT-PCR, PCR, hybridization or other means to determine the amount of nucleotides.

## Claims

1. An in vitro method for identifying from a biological fluid sample from a pregnant subject a pregnancy related syndrome selected from the group consisting of early-onset Preeclampsia, Eclampsia, and Hemolysis Elevated Liver enzymes and Low Platelets (HELLP), the method including (i) measuring the amount of ESM-1 in the biological sample, wherein the biological sample is from the pregnant subject being in any one of week 9-16 of gestation; (ii) comparing said amount of ESM-1 to a reference value, and (iii) identifying the subject as being likely to have or develop the pregnancy related syndrome based on a comparison of the amount of ESM-1 to the reference value, wherein said biological fluid sample comprises a blood sample, a plasma sample, or a serum sample from said pregnant subject.

2. The method according to claim 1, wherein the reference value is the amount of ESM-1 in biological samples of pregnant subjects having a healthy pregnancy.

3. The method according to any one of the preceding claims, wherein said biological fluid sample is from a pregnant subject being in any one week of gestation between weeks 12 to 16 of gestation, and wherein said biological fluid sample comprises a plasma sample and wherein the pregnant subject is evaluated to have the pregnancy related syndrome when the ESM-1 value is in the range of 410±355 pg/ml.

4. The method according to any one of the preceding claims, wherein the amount of ESM-1 of the biological fluid sample of the pregnant subject is indicative of the pregnancy related syndrome when the amount is equal to or less than 70% of the amount of ESM-1 of biological fluid samples of pregnant subjects having a healthy pregnancy.

5. The method according to any one of the preceding claims, further comprising using an assay specific to ESM-1, wherein the assay comprises an ELISA (Enzyme-linked immunosorbent assay) assay, a turbidimetric assay, radioimmunosorbent assay (RIST), a radioimmunoassay (RIA), a direct or indirect immunofluorescence assay, a particle gel immuno assay (PaGIA), or a lateral flow assay, especially wherein the assay comprises one or more of an ELISA, a lateral flow assay, an enzymatic assay, a colorimetric assay, and a luminescent assay, and/or wherein measuring the amount of ESM-1 in the biological sample includes using mass spectrometry (MS); the method further comprising the step of uterine artery Doppler screening and deriving thereof information about the placentation.

6. The method according to any one of the preceding claims, wherein said amount of ESM-1 is the level of free ESM-1 in the biological sample.

7. The method according to any one of the preceding claims, wherein one or more of (a) measuring the amount of ESM-1 in the biological sample comprises a method to do a quantification of RNA coding for ESM-1 in cells or cell-free RNA in the biological sample, and (b) measuring the amount of ESM-1 in the biological sample comprises a method to quantify the amount of protein via the amount of RNA coding for ESM-1; and wherein said quantification is done via RT-PCR, PCR, hybridization or other means to determine the amount of nucleotides.

8. The method according to any one of the preceding claims, further comprising (iv) using a secondary marker selected from the group consisting of soluble Fms-like tyrosine kinase-1 (sFlt1), Vascular Endothelial Growth Factor (VEGF), Placental Growth Factor (PlGF), Hepatocyte Growth Factor (HGF), soluble Endoglin, placental protein 13 (pp-13), Pregnancy- associated Plasma Protein A (PAPP-A) Growth Differentiation Factor 15 (GDF-15), pikachurin and a hemopexin, (v) optionally using an assay specific for the secondary marker, and measuring the amount of the secondary marker in the biological sample, and optionally comparing said amount of said secondary marker to a secondary marker reference value, the method further comprising identifying the subject as being likely to have or develop the pregnancy related syndrome based on a comparison of the amount of ESM-1 to a reference value, and based on a comparison of the amount of said one or more secondary markers with the corresponding secondary marker reference value, and the method further comprising determining a ratio of the amount of ESM-1 to the amount of the secondary marker, selecting a ratio reference value, and comparing the ratio with the ratio reference value, and the method further comprising identifying the subject as being likely to have or develop the pregnancy related syndrome based on a comparison of the amount of ESM-1 to a reference value, and based on a comparison of the ratio of the amount of ESM-1 to the amount of the secondary marker to the ratio reference value.

9. The method according to any one of the preceding claims, the method comprising providing a first biological sample from a subject extracted on a first occasion, and providing a second biological sample from the subject extracted on a second occasion, using an assay specific to ESM-1, measuring the amount of ESM-1 in the first biological sample, constituting a first amount, using an assay specific to ESM-1, measuring the amount of ESM-1 in the second biological sample, and constituting a second amount, and the method further comprising identifying the subject as being likely to have or develop the pregnancy related syndrome based on a comparison of the first amount and the second amount.

10. A device for identifying a pregnant subject being in any one of week 9-16 of gestation that is likely to have or develop a pregnancy related syndrome selected from the group consisting of early-onset Preeclampsia, Eclampsia, and HELLP, said device comprising (a) an analyzing unit comprising a detection agent for ESM-1, for instance being an antibody, a (recombinant) receptor for ESM-1 or an aptamer, which allows the determination of the amount of ESM-1; (b) an evaluation unit comprising a data processor having implemented necessary algorithms for comparing the amount of ESM-1 determined with a reference value stored in a database in order to determine whether a pregnant subject is likely to have or develop early-onset preeclampsia, eclampsia, and/or HELLP.

11. The device according to claim 10, further comprising an analyzing unit comprising a detection agent for sFlt1, VEGF, P1GF, HGF, sEndoglin, pp-13, PAPP-A or GDF-15, which allows the determination of the amount of sFlt1, VEGF, PlGF, HGF, sEndoglin, pp-13, PAPP-A or GDF-15, and an evaluation unit comprising a data processor having implemented necessary algorithms for comparing the amount of sFlt1, VEGF, PlGF, HGF, sEndoglin, pp-13, PAPP-A or GDF-15 determined with reference values stored in a database and calculate the ratio between the measured markers in order to determine whether a pregnant subject is likely to have or develop early-onset Preeclampsia, eclampsia, and/or HELLP, and wherein said evaluation unit is also capable of giving therapeutic recommendations.

12. Use of an evaluation of the amount of ESM-1 in an extracted biological fluid sample from a pregnant subject being in any one of week 9-16 of gestation for identifying whether the pregnant subject is likely to have or develop a pregnancy related syndrome selected from the group consisting of early-onset Preeclampsia, Eclampsia, and HELLP, wherein said biological fluid sample comprises a blood sample, a plasma sample, or a serum sample from said pregnant subject.

13. In vitro use of an extracted biological fluid sample from a pregnant subject being in any one of week 9-16 of gestation for identifying whether the pregnant subject is likely to have or develop a pregnancy related syndrome selected from the group consisting of early-onset Preeclampsia, Eclampsia, and HELLP, the use comprising: (a) measuring the amount of ESM-1 in the biological sample, especially using an assay specific to ESM-1; (b) comparing said amount of ESM-1 to a reference value; and (c) identifying the subject as being likely to have or develop the pregnancy related syndrome, wherein said biological fluid sample comprises a blood sample, a plasma sample, or a serum sample from said pregnant subject.

14. A kit for identifying whether a pregnant subject being in any one of week 9-16 of gestation is likely to have or develop a pregnancy related syndrome selected from the group consisting of early-onset Preeclampsia, Eclampsia, and HELLP, said kit comprising: (a) an analyzing unit comprising a detection agent for ESM-1; (b) an evaluation unit for determining whether a pregnant subject is likely to have or develop the pregnancy related syndrome, based on a result provided by the analyzing unit, the evaluation unit comprising a data processor having implemented necessary algorithms for comparing the amount ESM-1, determined with reference values stored in a database and to determine whether a pregnant subject is likely to have or develop the pregnancy related syndrome.

15. A kit for identifying whether a pregnant subject being in any one of week 9-16 of gestation is likely to have or develop a pregnancy related syndrome selected from the group consisting of early-onset Preeclampsia, Eclampsia, and HELLP, said kit comprising: (a) an analyzing unit comprising a detection agent for ESM-1 and one or more of sFlt1, VEGF, PlGF, HGF, sEndoglin, pp-13, PAPP-A and GDF-15; (b) an evaluation unit for determining whether a pregnant subject is likely to have or develop the pregnancy related syndrome, based on a result provided by the analyzing unit, the evaluation unit comprising a data processor having implemented necessary algorithms for comparing the amount ESM-1, and one or more of sFlt1, VEGF, PlGF, HGF, sEndoglin, pp-13, PAPP-A and GDF-15 determined with reference values stored in a database and calculate the ratio between the measured markers in order to determine whether a pregnant subject is likely to have or develop the pregnancy related syndrome

16. The kit according to any one of claims 14-15, further comprising a manual or a reference to a manual, wherein the manual includes instructions how to extract a biological fluid sample from a pregnant subject and/or how to use the analyzing unit and/or how to use the evaluation unit, wherein the manual includes information to extract biological fluid sample from a pregnant subject in any one of week 9-16 of gestation, especially weeks 12-16 of gestation, and wherein the evaluation unit comprises a color scheme, wherein the analyzing unit is configured to provide a color reaction wherein the color is dependent upon the amount of ESM-1 in an extracted biological sample from a pregnant subject, wherein said biological fluid sample comprises a blood sample, a plasma sample, or a serum sample from said pregnant subject.

17. The kit according to any one of claims 15-16, comprising
(i) an analyzing unit comprising a detection agent for ESM-1 and one or more of sFlt1, VEGF, PlGF, HGF, sEndoglin, pp-13, PAPP-A and GDF-15, especially the analyzing unit comprising a detection agent for ESM-1 and a detection agent for one or more of sFlt1, VEGF, PlGF, HGF, sEndoglin, pp-13, PAPP-A and GDF-15, which allows the determination of the amount of ESM-1, and one or more of sFlt1, VEGF, PlGF, HGF, sEndoglin, pp-13, PAPP-A or GDF-15, wherein
(a) the analyzing unit comprises an assay, especially wherein the analyzing unit comprises an ELISA (Enzyme-linked immunosorbent assay) assay, a turbidimetric assay, radio immunosorbent assay (RIST), radioimmunoassay (RIA), direct or indirect immunofluorescence, particle gel immuno assay (PaGIA), or a lateral flow assay; or
(b) the analyzing unit comprises a method to do a quantification of the RNA coding for ESM-1 in cells or cell-free RNA in the biological fluid sample; and
(ii) an evaluation unit comprising a data processor for determining whether a pregnant subject is likely to have or develop the pregnancy related syndrome, especially a data processor having implemented necessary algorithms for comparing the amount ESM-1, determined with reference values stored in a database in order to determine whether a pregnant subject is likely to have or develop the pregnancy related syndrome.

18. Use of a kit according to any one of claims 14-17, for predicting a pregnant subject in any one of week 9-16 of gestation to develop after week 20 early onset severe early-onset Preeclampsia, based on a measurement of the amount of ESM-1 in a biological fluid sample from the pregnant subject, wherein the biological fluid sample comprises a blood sample, a plasma sample, or a serum sample from said pregnant subject.

## Patentansprüche

1. In vitro-Verfahren zum Identifizieren, aus einer biologischen Fluidprobe von einem schwangeren Subjekt, eines mit einer Schwangerschaft in Zusammenhang stehenden Syndroms, ausgewählt aus der Gruppe bestehend aus früh einsetzender Präeklampsie, Eklampsie und Hämolyse, erhöhten Leberwerte und niedriger Blutplättchenanzahl (HELLP), das Verfahren enthaltend (i) Messen der Menge an ESM-1 in der biologischen Probe, wobei die biologische Probe von dem schwangeren Subjekt stammt, das in einer Schwangerschaftswoche 9-16 ist; (ii) Vergleichen der Menge an ESM-1 mit einem Referenzwert und (iii) Identifizieren, dass das Subjekt mit Wahrscheinlichkeit das mit einer Schwangerschaft in Zusammenhang stehende Syndrom hat oder entwickelt, basierend auf einem Vergleich der Menge an ESM-1 mit dem Referenzwert, wobei die biologische Fluidprobe eine Blutprobe, eine Plasmaprobe oder eine Serumprobe von dem schwangeren Subjekt umfasst.

2. Verfahren nach Anspruch 1, wobei der Referenzwert die Menge an ESM-1 in biologischen Proben von schwangeren Subjekten mit einer gesunden Schwangerschaft ist.

3. Verfahren nach einem der vorstehenden Ansprüche, wobei die biologische Fluidprobe von einem schwangeren Subjekt stammt, das in einer Schwangerschaftswoche zwischen Schwangerschaftswochen 12 bis 16 ist, und wobei die biologische Fluidprobe eine Plasmaprobe umfasst und wobei das schwangere Subjekt evaluiert wird, an dem mit einer Schwangerschaft in Zusammenhang stehenden Syndrom zu leiden, wenn der ESM-1-Wert im Bereich von 410 ± 355 pg/ml ist.

4. Verfahren nach einem der vorstehenden Ansprüche, wobei die Menge an ESM-1 der biologischen Fluidprobe des schwangeren Subjekts das mit einer Schwangerschaft in Zusammenhang stehende Syndrom anzeigt, wenn die Menge gleich oder kleiner 70% der Menge an ESM-1 von biologischen Fluidproben schwangerer Subjekte mit einer gesunden Schwangerschaft ist.

5. Verfahren nach einem der vorstehenden Ansprüche, weiter umfassend ein Verwenden eines Tests, der für ESM-1 spezifisch ist, wobei der Test einen ELISA-(enzymgebundenen Immunsorbenstest) Test, einen turbidimetrischen Test, einen Radioimmunsorbenstest (RIST), einen Radioimmuntest (RIA), einen direkten oder indirekten Immunfluoreszenztest, einen Partikel-Gelimmuntest (PaGIA) oder einen Lateralströmungstest umfasst, im Speziellen wobei der Test einen oder mehrere von einem ELISA, einem Lateralströmungstest, einem enzymatischen Test, einem kolorimetrischen Test und einem Lumineszenztest umfasst, und/oder wobei ein Messen der Menge an ESM-1 in der biologischen Fluidprobe ein Verwenden einer Massenspektrometrie (MS) umfasst, wobei das Verfahren weiter den Schritt eines Doppler-Screenings der uterinen Arterien und Ableiten von Informationen über die Plazentation daraus umfasst.

6. Verfahren nach einem der vorstehenden Ansprüche, wobei die Menge an ESM-1 der Pegel an freiem ESM-1 in der biologischen Probe ist.

7. Verfahren nach einem der vorstehenden Ansprüche, wobei eines oder mehrere von (a) Messen der Menge an ESM-1 in der biologischen Probe ein Verfahren umfasst, um eine Quantifizierung von RNA, die für ESM-1 in Zellen codiert, oder zellfreier RNA in der biologischen Probe vorzunehmen, und (b) Messen der Menge an ESM-1 in der biologischen Probe ein Verfahren umfasst, um die Menge an Protein über die Menge an RNA, die für ESM-1 codiert, zu quantifizieren; und wobei die Quantifizierung über RT-PCR, PCR, Hybridisierung oder ein anderes Mittel erfolgt, um die Menge an Nukleotiden zu bestimmen.

8. Verfahren nach einem der vorstehenden Ansprüche, weiter umfassend (iv) Verwenden eines sekundären Markers, der ausgewählt ist aus der Gruppe bestehend aus löslichen Fms-artigen Tyrosin-Kinase-1 (sFlt1), vaskulärem endothelialen Wachstumsfaktor (VEGF), Plazenta Wachstumsfaktor (PlGF), Hepatozytenwachstumsfaktor (HGF), löslichem Endoglin, Plazentaprotein 13 (pp-13), schwangerschaftsassoziiertem Plasmaprotein A (PAPP-A) Wachstumsdifferenzierungsfaktor 15 (GDF-15), Pikachurin und einem Hämopexin, (v) optional Verwenden eines Tests, der für den sekundären Marker spezifisch ist, und Messen der Menge des sekundären Markers in der biologischen Probe und optional Vergleichen der Menge des sekundären Markers mit einem Referenzwert für den sekundären Marker, wobei das Verfahren weiter ein Identifizieren einer Wahrscheinlichkeit, dass das Subjekts das schwangerschaftsbezogene Syndrom hat oder entwickelt, basierend auf einem Vergleich der Menge an ESM-1 mit einem Referenzwert und basierend auf einem Vergleich der Menge des einen oder der mehreren sekundären Marker mit dem entsprechenden Referenzwert für den sekundären Marker, und wobei das Verfahren weiter ein Bestimmen eines Verhältnisses der Menge an ESM-1 zu der Menge des sekundären Markers, Auswählen eines Verhältnisreferenzwerts und Vergleichen des Verhältnisses mit dem Verhältnisreferenzwert umfasst, und das Verfahren weiter ein Identifizieren einer Wahrscheinlichkeit, dass das Subjekts das schwangerschaftsbezogene Syndrom hat oder entwickelt, basierend auf einem Vergleich der Menge an ESM-1 mit einem Referenzwert und basierend auf einem Vergleich des Verhältnisses der Menge an ESM-1 zu der Menge des sekundären Markers mit dem Verhältnisreferenzwert umfasst.

9. Verfahren nach einem der vorstehenden Ansprüche, das Verfahren umfassend ein Bereitstellen einer ersten biologischen Probe von einem Subjekt, die bei einer ersten Gelegenheit entnommen wurde, und Bereitstellen einer zweiten biologischen Probe von dem Subjekt, die bei einer zweiten Gelegenheit entnommen wurde, Verwenden eines für ESM-1 spezifischen Tests, Messen der Menge an ESM-1 in der ersten biologischen Probe, die eine erste Menge darstellt, unter Verwendung eines Tests, der für ESM-1 spezifisch ist, Messen der Menge an ESM-1 in der zweiten biologischen Probe, und die eine zweite Menge darstellt, und das Verfahren weiter umfassend ein Identifizieren einer Wahrscheinlichkeit, dass das Subjekts das schwangerschaftsbezogene Syndrom hat oder entwickelt, basierend auf einem Vergleich der ersten Menge und der zweiten Menge.

10. Vorrichtung zum Identifizieren einer Wahrscheinlichkeit, dass ein schwangeres Subjekt, das in einer Schwangerschaftswoche 9-16 ist, ein schwangerschaftsbezogenes Syndrom hat oder entwickelt, ausgewählt aus der Gruppe bestehend aus früh einsetzender Präeklampsie, Eklampsie und HELLP, wobei die Vorrichtung (a) eine Analysiereinheit, die ein Detektionsmittel für ESM-1 umfasst, das beispielsweise ein Antikörper, ein (rekombinanter) Rezeptor für ESM-1 oder ein Aptamer ist, das die Bestimmung der Menge an ESM-1 erlaubt; (b) eine Evaluierungseinheit, die einen Datenprozessor, in dem notwendige Algorithmen für einen Vergleich der bestimmten Menge an ESM-1 mit einem Referenzwert, der in einer Datenbank gespeichert ist, implementiert sind, um eine Wahrscheinlichkeit zu bestimmen, dass ein schwangeres Subjekt an früh einsetzender Präeklampsie, Eklampsie und/oder HELLP leidet, umfasst.

11. Vorrichtung nach Anspruch 10, weiter umfassend eine Analysiereinheit, die ein Detektionsmittel für sFlt1, VEGF, PlGF, HGF, sEndglin, pp-13, PAPP-A oder GDF-15 umfasst, das die Bestimmung der Menge an sFlt1, VEGF, PlGF, HGF, sEndglin, pp-13, PAPP-A oder GDF-15 erlaubt, und eine Evaluierungseinheit, die einen Datenprozessor, im dem notwendige Algorithmen für einen Vergleich der bestimmten Menge an sFlt1, VEGF, PlGF, HGF, sEndglin, pp-13, PAPP-A oder GDF-15 mit Referenzwerten, die in einer Datenbank gespeichert sind, implementiert sind, und der das Verhältnis zwischen den gemessenen Markern berechnet, um eine Wahrscheinlichkeit zu bestimmen, dass ein schwangeres Subjekt an früh einsetzender Präeklampsie, Eklampsie und/oder HELLP leidet, und wobei die Evaluierungseinheit auch imstande ist, therapeutische Empfehlungen zu geben.

12. Verwendung einer Evaluierung der Menge an ESM-1 in einer entnommenen biologischen Fluidprobe von einem schwangeren Subjekt, das in einer Schwangerschaftswoche 9-16 ist, zum Identifizieren einer Wahrscheinlichkeit, dass das schwangere Subjekt ein schwangerschaftsbezogenes Syndrom hat oder entwickelt, ausgewählt aus der Gruppe bestehend aus früh einsetzender Präeklampsie, Eklampsie und HELLP, wobei die biologische Fluidprobe eine Blutprobe, eine Plasmaprobe oder eine Serumprobe von dem schwangeren Subjekt umfasst.

13. In-vitro-Verwendung einer gewonnenen biologischen Fluidprobe von einem schwangeren Subjekt, das in einer Schwangerschaftswoche 9-16 ist, zum Identifizieren einer Wahrscheinlichkeit, dass das schwangere Subjekt ein schwangerschaftsbezogenes Syndrom hat oder entwickelt, ausgewählt aus der Gruppe bestehend aus früh einsetzender Präeklampsie, Eklampsie und HELLP, die Verwendung umfassend (a) Messen der Menge an ESM-1 in der biologischen Probe, im Speziellen unter Verwendung eines Tests, der für ESM-1 spezifisch ist; (b) Vergleichen der Menge an ESM-1 mit einem Referenzwert; und (c) Identifizieren, dass das Subjekt mit Wahrscheinlichkeit das mit einer Schwangerschaft in Zusammenhang stehende Syndrom hat oder entwickelt, wobei die biologische Fluidprobe eine Blutprobe, eine Plasmaprobe oder eine Serumprobe von dem schwangeren Subjekt umfasst.

14. Kit zum Identifizieren einer Wahrscheinlichkeit, dass ein schwangeres Subjekt, das in einer Schwangerschaftswoche 9-16 ist, ein schwangerschaftsbezogenes Syndrom hat oder entwickelt, ausgewählt aus der Gruppe bestehend aus früh einsetzender Präeklampsie, Eklampsie und HELLP, wobei das Kit umfasst: (a) eine Analysiereinheit, die ein Detektionsmittel für ESM-1 umfasst; (b) eine Evaluierungseinheit zum Bestimmen einer Wahrscheinlichkeit, dass ein schwangeres Subjekt das schwangerschaftsbezogene Syndrom hat oder entwickelt, basierend auf einem Ergebnis, das durch die Analysiereinheit bereitgestellt wird, wobei die Evaluierungseinheit einen Datenprozessor umfasst, in dem notwendige Algorithmen für einen Vergleich der bestimmten Menge an ESM-1 mit Referenzwerten, die in einer Datenbank gespeichert sind, implementiert sind und der eine Wahrscheinlichkeit bestimmt, dass ein schwangeres Subjekt das schwangerschaftsbezogene Syndrom hat oder entwickelt.

15. Kit zum Identifizieren einer Wahrscheinlichkeit, dass ein schwangeres Subjekt, das in einer Schwangerschaftswoche 9-16 ist, ein schwangerschaftsbezogenes Syndrom hat oder entwickelt, ausgewählt aus der Gruppe bestehend aus früh einsetzender Präeklampsie, Eklampsie und HELLP, wobei das Kit umfasst: (a) eine Analysiereinheit, die ein Detektionsmittel für ESM-1 und eines oder mehrere von sFlt1, VEGF, PlGF, HGF, sEndglin, pp-13, PAPP-A und GDF-15 umfasst; (b) eine Evaluierungseinheit zum Bestimmen einer Wahrscheinlichkeit, dass ein schwangeres Subjekt das schwangerschaftsbezogene Syndrom hat oder entwickelt, basierend auf einem Ergebnis, das durch die Analysiereinheit bereitgestellt wird, wobei die Evaluierungseinheit einen Datenprozessor umfasst, in dem notwendige Algorithmen für einen Vergleich der bestimmten Menge an ESM-1 und einem oder mehreren von sFlt1, VEGF, PlGF, HGF, sEndglin, pp-13, PAPP-A und GDF-15 mit Referenzwerten, die in einer Datenbank gespeichert sind, implementiert sind und der das Verhältnis zwischen den gemessenen Markern berechnet, um eine Wahrscheinlichkeit zu bestimmen, dass ein schwangeres Subjekt das schwangerschaftsbezogene Syndrom hat oder entwickelt.

16. Kit nach einem der Ansprüche 14-15, weiter umfassend ein Handbuch oder einen Verweis auf ein Handbuch, wobei das Handbuch Anweisungen enthält, wie eine biologische Fluidprobe einem schwangeren Subjekt zu entnehmen und/oder wie die Analysiereinheit zu verwenden ist und/oder wie die Evaluierungseinheit zu verwenden ist, wobei das Handbuch Informationen zum Entnehmen einer biologischen Fluidprobe von einem schwangeren Subjekt in einer Schwangerschaftswoche 9-16, im Speziellen in Schwangerschaftswochen 12-16, enthält, und wobei die Evaluierungseinheit ein Farbschema umfasst, wobei die Analysiereinheit konfiguriert ist, eine Farbreaktion bereitzustellen, wobei die Farbe von der Menge an ESM-1 in einer einem schwangeren Subjekt entnommenen biologischen Probe abhängig ist, wobei die biologische Fluidprobe eine Blutprobe, eine Plasmaprobe oder eine Serumprobe von dem schwangeren Subjekt umfasst.

17. Kit nach einem der Ansprüche 15-16, umfassend
(i) eine Analysiereinheit, die ein Detektionsmittel für ESM-1 und eines oder mehrere von sFlt1, VEGF, PlGF, HGF, sEndglin, pp-13, PAPP-A und GDF-15 umfasst, im Speziellen die Analysiereinheit, die ein Detektionsmittel für ESM-1 und ein Detektionsmittel für eines oder mehrere von sFlt1, VEGF, PlGF, HGF, sEndglin, pp-13, PAPP-A und GDF-15 umfasst, das eine Bestimmung der Menge an ESM-1 und eines oder mehrerer von sFlt1, VEGF, PlGF, HGF, sEndglin, pp-13, PAPP-A oder GDF-15 erlaubt, wobei
(a) die Analysiereinheit einen Test umfasst, wobei die Analysiereinheit im speziellen einen ELISA- (enzymgebundenen Immunsorbenstest) Test, einen turbidimetrischen Test, einen Radioimmunsorbenstest (RIST), einen Radioimmuntest (RIA), einen direkten oder indirekten Immunfluoreszenztest, einen Partikel-Gelimmuntest (PaGIA) oder einen Lateralströmungstest umfasst; oder
(b) die Analysiereinheit ein Verfahren zum Vornehmen einer Quantifizierung der RNA, die für ESM-1 in Zellen codiert, oder einer zellfreien RNA in der biologischen Fluidprobe umfasst; und
(ii) eine Evaluierungseinheit, die einen Datenprozessor zum Bestimmen einer Wahrscheinlichkeit umfasst, dass ein schwangeres Subjekt das schwangerschaftsbezogene Syndrom hat oder entwickelt, im Speziellen einen Datenprozessor, in dem notwendige Algorithmen für einen Vergleich der bestimmten Menge an ESM-1 mit Referenzwerten, die in einer Datenbank gespeichert sind, implementiert sind, um eine Wahrscheinlichkeit zu bestimmen, dass ein schwangeres Subjekt das schwangerschaftsbezogene Syndrom hat oder entwickelt.

18. Verwendung eines Kits nach einem der Ansprüche 14-17 zur Vorhersage, ob ein schwangeres Subjekt in einer Schwangerschaftswoche 9-16 nach Woche 20 früh einsetzende schwere, früh einsetzende Präeklampsie entwickeln wird, basierend auf einer Messung der Menge an ESM-1 in einer biologischen Fluidprobe von dem schwangeren Subjekt, wobei die biologische Fluidprobe eine Blutprobe, eine Plasmaprobe oder eine Serumprobe von dem schwangeren Subjekt umfasst.

## Revendications

1. Procédé in vitro pour identifier à partir d'un échantillon de fluide biologique provenant d'un sujet enceinte un syndrome lié à la grossesse sélectionné dans le groupe constitué par l'Éclampsisme à début précoce, l'Éclampsie et Hémolyse, Tests du Foie Élevés, Plaquettes Basses (HTFEPB), le procédé incluant
(i) la mesure de la quantité d'ESM-1 dans l'échantillon biologique, dans lequel l'échantillon biologique provient du sujet enceinte qui est dans l'une quelconque des semaines 9 à 16 de gestation ;
(ii) la comparaison de ladite quantité d'ESM-1 à une valeur de référence, et
(iii) l'identification du sujet comme étant susceptible d'avoir ou de développer le syndrome lié à la grossesse sur la base d'une comparaison de la quantité d'ESM-1 à la valeur de référence, dans lequel ledit échantillon de fluide biologique comprend un échantillon de sang, un échantillon de plasma, ou un échantillon de sérum provenant dudit sujet enceinte.

2. Procédé selon la revendication 1, dans lequel la valeur de référence est la quantité d'ESM-1 dans des échantillons biologiques de sujets enceintes ayant une grossesse saine.

3. Procédé selon l'une quelconque des revendications précédentes, dans lequel ledit échantillon de fluide biologique provient d'un sujet enceinte qui est dans l'une quelconque semaine de gestation entre les semaines 12 à 16 de gestation, et dans lequel ledit échantillon de fluide biologique comprend un échantillon de plasma et dans lequel le sujet enceinte est évalué comme ayant le syndrome lié à la grossesse quand la valeur d'ESM-1 est dans la plage de 410 ± 355 pg/ml.

4. Procédé selon l'une quelconque des revendications précédentes, dans lequel la quantité d'ESM-1 de l'échantillon de fluide biologique du sujet enceinte est indicative du syndrome lié à la grossesse quand la quantité est égale ou inférieure à 70% de la quantité d'ESM-1 des échantillons de fluide biologique de sujets enceintes ayant une grossesse saine.

5. Procédé selon l'une quelconque des revendications précédentes, comprenant en outre l'utilisation d'un titrage spécifique à ESM-1, dans lequel le titrage comprend un titrage ELISA (titrage immunoenzymatique utilisant un antigène absorbé), un titrage turbidimétrique, un radio-immuno-sorbent test (RIST), une radio-immuno-analyse (RIA), un titrage par immunofluorescence directe ou indirecte, un immunoessai sur gel de particules (PaGIA), ou un titrage à flux latéral, particulièrement dans lequel le titrage comprend un ou plusieurs d'un ELISA, d'un titrage à flux latéral, d'un titrage enzymatique, d'un titrage colorimétrique et d'un titrage luminescent, et/ou dans lequel la mesure de la quantité d'ESM-1 dans l'échantillon biologique inclut l'utilisation d'une spectrométrie de masse (MS) ; le procédé comprenant en outre l'étape criblage Doppler de l'artère utérine et l'obtention à partir de celle-ci d'informations concernant la placentation.

6. Procédé selon l'une quelconque des revendications précédentes, dans lequel ladite quantité d'ESM-1 est le niveau d'ESM-1 libre dans l'échantillon biologique.

7. Procédé selon l'une quelconque des revendications précédentes, dans lequel une ou plusieurs (a) d'une mesure de la quantité d'ESM-1 dans l'échantillon biologique comprend un procédé pour effectuer une quantification d'ARN codant pour ESM-1 dans des cellules ou d'ARN sans cellule dans l'échantillon biologique, et (b) d'une mesure de la quantité d'ESM-1 dans l'échantillon biologique comprend un procédé pour quantifier la quantité de protéine via la quantité d'ARN codant pour ESM-1 ; et dans lequel ladite quantification est faite par RT-PCR, PCR, hybridation ou autre moyen pour déterminer la quantité de nucléotides.

8. Procédé selon l'une quelconque des revendications précédentes, comprenant en outre (iv) l'utilisation d'un marqueur secondaire sélectionné dans le groupe constitué par la tyrosine kinase-1 semblable à Fms soluble (sFlt1), le Facteur de Croissance de l'Endothélium Vasculaire (VEGF), le Facteur de Croissance Placentaire (PIGF), le Facteur de Croissance de Cellule Hépatique (HGF), l'Endogline soluble, la protéine placentaire 13 (pp-13), le Facteur 15 de Différentiation de Croissance (GDF-15) de la Protéine de Plasma associée à la Grossesse (PAPP-A), la pikachurine et une hémopexine, (v) de manière facultative l'utilisation d'un titrage spécifique pour le marqueur secondaire, et la mesure de la quantité du marqueur secondaire dans l'échantillon biologique, et de manière facultative la comparaison de ladite quantité de marqueur secondaire à une valeur de référence de marqueur secondaire, le procédé comprenant en outre l'identification du sujet comme étant susceptible d'avoir ou de développer le syndrome lié à la grossesse sur la base d'une comparaison de la quantité d'ESM-1 à une valeur de référence, et sur la base d'une comparaison de la quantité desdits un ou plusieurs marqueurs secondaires à la valeur de référence de marqueur secondaire correspondante, et le procédé comprenant en outre la détermination d'un rapport de la quantité d'ESM-1 sur la quantité du marqueur secondaire, la sélection d'une valeur de référence de rapport, et la comparaison du rapport à la valeur de référence de rapport, et le procédé comprenant en outre l'identification du sujet comme étant susceptible d'avoir ou de développer le syndrome lié à la grossesse sur la base d'une comparaison de la quantité d'ESM-1 à une valeur de référence, et sur la base d'une comparaison du rapport de la quantité d'ESM-1 sur la quantité du marqueur secondaire à la valeur de référence de rapport.

9. Procédé selon l'une quelconque des revendications précédentes, le procédé comprenant la fourniture d'un premier échantillon biologique provenant d'un sujet, extrait lors d'une première occasion, et la fourniture d'un second échantillon biologique provenant du sujet, extrait lors d'une seconde occasion, l'utilisation d'un titrage spécifique à ESM-1, la mesure de la quantité d'ESM-1 dans le premier échantillon biologique, la constitution d'une première quantité, l'utilisation d'un titrage spécifique à ESM-1, la mesure de la quantité d'ESM-1 dans le second échantillon biologique, et la constitution d'une seconde quantité, et le procédé comprenant en outre l'identification du sujet comme étant susceptible d'avoir ou de développer le syndrome lié à la grossesse sur la base d'une comparaison de la première quantité et de la seconde quantité.

10. Dispositif pour identifier un sujet enceinte qui est dans l'une quelconque des semaines 9 à 16 de gestation qui est susceptible d'avoir ou de développer un syndrome lié à la grossesse sélectionné dans le groupe constitué par l'Éclampsisme à début précoce, l'Éclampsie et HTFEPB, ledit dispositif comprenant (a) une unité d'analyse comprenant un agent de détection pour ESM-1, qui est par exemple un anticorps, un récepteur (recombinant) pour ESM-1 ou un aptamère, qui permet la détermination de la quantité d'ESM-1 ; (b) une unité d'évaluation comprenant un processeur de données ayant des algorithmes nécessaires implémentés pour comparer la quantité d'ESM-1 déterminée à une valeur de référence stockée dans une base de données afin de déterminer si un sujet enceinte est susceptible d'avoir ou de développer un éclampsisme à début précoce, une éclampsie, et/ou HTFEPB.

11. Dispositif selon la revendication 10, comprenant en outre une unité d'analyse comprenant un agent de détection pour sFlt1, VEGF, PlGF, HGF, sEndogline, pp-13, PAPP-A ou GDF-15, qui permet la détermination de la quantité de sFlt1, VEGF, PlGF, HGF, sEndogline, pp-13, PAPP-A ou GDF-15, et une unité d'évaluation comprenant un processeur de données ayant des algorithmes nécessaires implémentés pour comparer la quantité de sFlt1, VEGF, PlGF, HGF, sEndogline, pp-13, PAPP-A ou GDF-15 déterminée à des valeurs de référence stockées dans une base de données et pour calculer le rapport entre les marqueurs mesurés pour déterminer si un sujet enceinte est susceptible d'avoir ou de développer un Éclampsisme à début précoce, une éclampsie, et/ou HTFEPB, et dans lequel ladite unité d'évaluation est également susceptible de donner des recommandations thérapeutiques.

12. Utilisation d'une évaluation de la quantité d'ESM-1 dans un échantillon de fluide biologique extrait provenant d'un sujet enceinte qui est dans l'une quelconque des semaines 9 à 16 de gestation pour identifier si le sujet enceinte est susceptible d'avoir ou de développer un syndrome lié à la grossesse sélectionné dans le groupe constitué par l'Éclampsisme à début précoce, l'Éclampsie et HTFEPB, dans lequel ledit échantillon de fluide biologique comprend un échantillon de sang, un échantillon de plasma, ou un échantillon de sérum dudit sujet enceinte.

13. Utilisation in vitro d'un échantillon de fluide biologique extrait provenant d'un sujet enceinte qui est dans l'une quelconque des semaines 9 à 16 de gestation pour identifier si le sujet enceinte est susceptible d'avoir ou de développer un syndrome lié à la grossesse sélectionné dans le groupe constitué de l'Éclampsisme à début précoce, l'Éclampsie et HTFEPB, l'utilisation comprenant : (a) la mesure de la quantité d'ESM-1 dans l'échantillon biologique, particulièrement en utilisant un titrage spécifique à ESM-1;(b) la comparaison de ladite quantité d'ESM-1 à une valeur de référence; et (c) l'identification du sujet comme étant susceptible d'avoir ou de développer le syndrome lié à la grossesse, dans lequel ledit échantillon de fluide biologique comprend un échantillon de sang, un échantillon de plasma, ou un échantillon de sérum dudit sujet enceinte.

14. Kit pour identifier si un sujet enceinte qui est dans l'une quelconque des semaines 9 à 16 de gestation est susceptible d'avoir ou de développer un syndrome lié à la grossesse sélectionné dans le groupe constitué par l'Éclampsisme à début précoce, l'Éclampsie et HTFEPB, ledit kit comprenant : (a) une unité d'analyse comprenant un agent de détection pour ESM-1 ; (b) une unité d'évaluation pour déterminer si un sujet enceinte est susceptible d'avoir ou de développer le syndrome lié à la grossesse, sur la base d'un résultat fourni par l'unité d'analyse, l'unité d'évaluation comprenant un processeur de données ayant des algorithmes nécessaires implémentés pour comparer la quantité d'ESM-1 déterminée à des valeurs de référence stockées dans une base de données et pour déterminer si un sujet enceinte est susceptible d'avoir ou de développer le syndrome lié à la grossesse.

15. Kit pour identifier si un sujet enceinte qui est dans l'une quelconque des semaines 9 à 16 de gestation est susceptible d'avoir ou de développer un syndrome lié à la grossesse sélectionné dans le groupe constitué par l'Éclampsisme à début précoce, l'Éclampsie et HTFEPB, ledit kit comprenant : (a) une unité d'analyse comprenant un agent de détection pour ESM-1 et un ou plusieurs de sFlt1, VEGF, PlGF, HGF, sEndogline, pp-13, PAPP-A et GDF-15 ; (b) une unité d'évaluation pour déterminer si un sujet enceinte est susceptible d'avoir ou de développer le syndrome lié à la grossesse, sur la base d'un résultat fourni par l'unité d'analyse, l'unité d'évaluation comprenant un processeur de données ayant des algorithmes nécessaires implémentés pour comparer la quantité d'ESM-1 et un ou plusieurs de sFlt1, VEGF, PlGF, HGF, sEndogline, pp-13, PAPP-A et GDF-15 déterminés à des valeurs de référence stockées dans une base de données et pour calculer le rapport entre les marqueurs mesurés afin de déterminer si un sujet enceinte est susceptible d'avoir ou de développer le syndrome lié à la grossesse.

16. Kit selon l'une quelconque des revendications 14 à 15, comprenant en outre un manuel ou une référence à un manuel, dans lequel le manuel inclut des instructions sur comment extraire un échantillon de fluide biologique provenant d'un sujet enceinte et/ou comment utiliser l'unité d'analyse et/ou comment utiliser l'unité d'évaluation, dans lequel le manuel inclut des informations pour extraire un échantillon de fluide biologique provenant d'un sujet enceinte qui est dans l'une quelconque des semaines 9 à 16 de gestation, particulièrement les semaines 12 à 16 de gestation, et dans lequel l'unité d'évaluation comprend une combinaison de couleurs, dans lequel l'unité d'analyse est configurée pour fournir une réaction colorée dans laquelle la couleur est dépendante de la quantité d'ESM-1 dans un échantillon biologique extrait provenant d'un sujet enceinte, dans lequel ledit échantillon de fluide biologique comprend un échantillon de sang, un échantillon de plasma, ou un échantillon de sérum provenant dudit sujet enceinte.

17. Kit selon l'une quelconque des revendications 15 à 16, comprenant
(i) une unité d'analyse comprenant un agent de détection pour ESM-1 et un ou plusieurs de sFlt1, VEGF, PlGF, HGF, sEndogline, pp-13, PAPP-A et GDF-15, particulièrement l'unité d'analyse comprenant un agent de détection pour ESM-1 et un agent de détection pour un ou plusieurs de sFlt1, VEGF, PlGF, HGF, sEndogline, pp-13, PAPP-A et GDF-15, qui permet la détermination de la quantité d'ESM-1 et d'un ou plusieurs de sFlt1, VEGF, PlGF, HGF, sEndogline, pp-13, PAPP-A ou GDF-15, dans lequel
(a) l'unité d'analyse comprend un titrage, particulièrement dans lequel l'unité d'analyse comprend un titrage ELISA (titrage immunoenzymatique utilisant un antigène absorbé), un titrage turbidimétrique, un radio-immuno-sorbent test (RIST), une radio-immuno-analyse (RIA), une immunofluorescence directe ou indirecte, un immunoessai sur gel de particules (PaGIA), ou un titrage à flux latéral; ou
(b) l'unité d'analyse comprend un procédé pour effectuer une quantification de l'ARN codant pour ESM-1 dans des cellules ou de l'ARN sans cellule dans l'échantillon de fluide biologique ; et
(ii) une unité d'évaluation comprenant un processeur de données pour déterminer si un sujet enceinte est susceptible d'avoir ou de développer le syndrome lié à la grossesse, particulièrement un processeur de données ayant des algorithmes nécessaires implémentés pour comparer la quantité d'ESM-1 déterminée à des valeurs de référence stockées dans une base de données pour déterminer si un sujet enceinte est susceptible d'avoir ou de développer le syndrome lié à la grossesse.

18. Utilisation d'un kit selon l'une quelconque des revendications 14 à 17, pour prévoir qu'un sujet enceinte dans l'une quelconque des semaines 9 à 16 de gestation va développer après la semaine 20 un Éclampsisme à début précoce sévère à début précoce, sur la base d'une mesure de la quantité d'ESM-1 dans un échantillon de fluide biologique provenant du sujet enceinte, dans lequel l'échantillon de fluide biologique comprend un échantillon de sang, un échantillon de plasma, ou un échantillon de sérum provenant dudit sujet enceinte.
